# EUROPÄISCHE PATENTSCHRIFT

(11) **EP 1 297 164 B1**
(45) Veröffentlichungstag und Bekanntmachung des Hinweises auf die Patenterteilung: **26.09.2007**
(21) Anmeldenummer: 01955328.8
(22) Anmeldetag: 03.07.2001
(51) Int. Cl.: C12N 15/82, C12N 5/10, A01H 5/00

(54) **PROMOTOREN ZUR GENEXPRESSION IN KARYOPSEN VON PFLANZEN**
PROMOTERS OF GENE EXPRESSION IN PLANT CARYOPSES
PROMOTEURS D'EXPRESSION GENIQUE DANS DES CARYOPSES DE PLANTES

(30) Priorität: 06.07.2000 DE 10032379; 26.08.2000 DE 10041861
(43) Veröffentlichungstag der Anmeldung: 02.04.2003
(73) Patentinhaber: Bayer CropScience AG, 40789 Monheim (DE)
(72) Erfinder: SPRUNCK, Stefanie, 93051 Regensburg (DE); KLUTH, Antje, 21465 Wentorf (DE); BECKER, Dirk, 21149 Hamburg (DE); LUETTICKE, Stephanie, 20099 Hamburg (DE); LOERZ, Horst, 22589 Hamburg (DE)
(74) Vertreter: Beyer, Andreas
(86) Internationale Anmeldenummer: PCT/EP2001/007592
(87) Internationale Veröffentlichungsnummer: WO 2002/002785

(56) Entgegenhaltungen:
- WO-A-97/45545
- WO-A-99/14314
- WO-A-99/40209
- US-A- 6 013 862
- AINSWORTH C ET AL: "EXPRESSION, ORGANISATION AND STRUCTURE OF THE GENES ENCODING THE WAXY PROTEIN (GRANULE-BOUND STARCH SYNTHASE) IN WHEAT" TRENDS IN BIOTECHNOLOGY, ELSEVIER PUBLICATIONS, CAMBRIDGE, GB, Bd. 22, 1993, Seiten 67-82, XP000996264 ISSN: 0167-7799
- DENYER K ET AL: "IDENTIFICATION OF MULTIPLE ISOFORMS OF SOLUBLE AND GRANULE-BOUND STARCH SYNTHASE IN DEVELOPING WHEAT ENDOSPERM" PLANTA, SPRINGER VERLAG, DE, Bd. 196, 1995, Seiten 256-265, XP002043126 ISSN: 0032-0935
- MURAI J ET AL: "Isolation and characterization of the three Waxy genes encoding the granule-bound starch synthase in hexaploid wheat" GENE, ELSEVIER BIOMEDICAL PRESS. AMSTERDAM, NL, Bd. 234, Nr. 1, 24. Juni 1999 (1999-06-24), Seiten 71-79, XP004176890 ISSN: 0378-1119
- DATABASE EMBL SEQUENCE LIBRARY [Online] 20. Februar 1989 (1989-02-20) RHODE, W., ET AL. : "structural analysis of the waxy locus from Hordeum vulgare" XP002183957 in der Anmeldung erwähnt
- DATABASE EMBL SEQUENCE DATABASE [Online] 4. Januar 2001 (2001-01-04) CLARKE, ET AL. : "Genes active in developing wheat endosperm - unpublished; BRY_1580 BRY Triticum aestivum cDNA clone P56-1P, mRNA sequence" XP002183958

### SEQUENCE LISTING

<110> Bayer CropScience GmbH
<120> Promotor zur Genexpression in Karyopsen von Pflanzen
<130> AGR 2000/M 226
<140> -
   <141> 2000-08-25
<160> 10
<170> PatentIn Ver. 2.1
<210> 1
   <211> 3785
   <212> DNA
   <213> Triticum aestivum
<400> 1
   gtttggtttc gctgtttttc atttcctttc ttcttaaggg gtaataccaa tgacagtaat 60
   tcatattgtg taacagtgcg attcttgtgc caattatgta caatttcttt tgtaattgtt 120
   tgtttcatgt tttatttcat tttctttact ttttagggta aaaccaatgc ccccaattca 180
   ttctacctaa gaggaaattc agttttatac tagtttcagt tttattattg tttattaagt 240
   gtttttagtt ggttttctca tttatgtgta tgcatgaata ttaggggtgt gtgtgcgtgt 300
   gttaatatac acataagtat tatacaccca tttttgcagt cataaaatta tgcaatttca 360
   gtacaaattg tgcgcaaact cttcttcatt ttttattttt tattttattt tcttctttaa 420
   gggtaatacc aatgatacta atttatgcct catttggaaa tttcgttttg aaaattatgc 480
   tagtacacac ttattcttgt atattatgga aaagcgcaat ttctgtgtaa gttttgtcat 540
   tctgtatttt ttttcatttt tctttcttct ggaagggtaa cactaatgcc actaattcat 600
   tcttgcttag aaaactttag tattttgatt gtgttttagt ttttatttca ttttgtttct 660
   tctttaaggg aaataccaat gccactaatc cattccatct tagaaaatct ctttatctta 720
   caaaaactca acttttatat gcttattcgt gcatattata aaaagcacag tttctatcta 780
   aattgcgtgc aaactttatc attatttgtc taaattaatt ttttctagaa tgatgatacc 840
   aatgccacta attcattccg tgagcacgca atatgcggaa tgcctacgta tattagtggt 900
   gtcgcatttt tcatctctca cgcatgggca tgcataccct acacatgcac acacacgcat 960
   acacaacaca tgagcactca cgcgagcaca tgcatacacc tgtgcgcaca cacacgacac 1020
   cgacacacac gcacagccac atgcgtgcac ttagaaagaa aaaatagaca cgtatacatt 1080
   ggactggcta gctatactac cgtgtaacac tagtacgttg gtgttgtacg acctattttc 1140
   aggtgccaca gactagtatt ttcaggcgac tgggatatag ccacggccta ttgtttcgtg 1200
   tcgtaggacg aaaacggtca tatatgtggc actggccttc tagagactct ccaagaggct 1260
   caccacctca ccgtgagtga cagcccaccg tcgcgtaaac caccgcattt acgtttcccc 1320
   gatccgacaa agccagggca cgcacgtacg tgtccatgtt ggcacgtgcg tgcgtccctc 1380
   acgcgccggt ttggcagcac gtacgtgcta gctgttcata ccagagccgt acgtcaatca 1440
   agcaaaagag aaaaagaagg ggcgaaaggt gatacgcccg gccgtgtcgt cgtgctgcag 1500
   aggaagcaat cccgggccat gcagcgccat tgccacgccc cagcgaaaag cgaaggcgag 1560
   agcgagagca cacatggccc ccagaactga aagcgaggga gcacacgaga aggcgcgtgc 1620
   gcgtggacat cacagcagga acacccaccg gcagcccacc gggcgggcgc gggcaggaca 1680
   agaagatgcg tgcacggcgc ggccggcaac ggaagggggc gccgccggcc gagcgcacgc 1740
   aaaggcgcgt cggccagcca cgacgccgct ggaaagcgcg ccggcgaacc gagaatgtgc 1800
   caggctgcca gccgctccgc ggtaccacta gtctcgtacg tgtgccactc cactccgctc 1860
   cgctcggcac gcacgcacgc aggcagaaac aaacaaacaa acaaaaaagt gggtcattca 1920
   ctccactcaa cgtcgccttt caggacgatg cttcggtgcc ttaagacacc tacctttgtg 1980
   tctatgacat gtgagcccaa cagatggctg gcccacatgt cagtgatcca aaggcaggtg 2040
   cctttaaagc accgaagctg cgtcccgcct ttcattacac gggccatgca tgcgggtgcg 2100
   tgccgtcccg tctaggcgtt cggtgtccgg ccgcgtgcat gcatgcacga ggagcggagc 2160
   ggagcgggta ttggggatcc agccaccgga ggactgagcg agcgggcgag tacaaataac 2220
   cccactcacc ggagccacgc accgttcgtt tccttgagtc ccgtcacttt cgcccgcccg 2280
   ccccacacac tacaaccagg agcctcgatc tgccagtgaa gaagaagaag gacactcacg 2340
   aatgcccggc cggcgactgt gagtacgctc ccgtccagga agaagaagaa gaagaagaag 2400
   cagaagaaga agaagcagaa gaagagatca gaccaggtac gcacgaacgt atatagtcag 2460
   gccggcccag ttcccggccg ccggacgatg gatagatcga tttagttcgg tctcaaatca 2520
   aggtcggttg gtctagtagt agatagatcc atccaaatgc cgccatgttg ttagatccag 2580
   agtctcttcc tttttactta aagatcgcga gcgtaagttg aggatcttcc tatagattcg 2640
   tagatttaaa atcatgtaaa aattaaaaaa aaagatttaa aatcatgtac tgctagctag 2700
   gatggatttc tatgtgaacg atcttagatc tgcggaacag atccaatgga ttcatggccg 2760
   gcctagggtt aattacgact agacagaggc agcataatgc gcgcataaac atttctgttt 2820
   tctagccgag ttggatcaaa caggtcaggt cacgcaccaa ggctttgatt tttgtttgtt 2880
   tttggcgtgg gcgttccact gcaccctaca gaacaaattc catttctcag ccagttccac 2940
   cccgtgcacg cgatttaaca gcttattaat tactaccagt gcggagacag gttcatatat 3000
   actctggtca tgttaatttg gatttcaaat tcaaatgtaa aatccagaaa acttgactgc 3060
   aaattctggt ttacttcact actcactaac aatcagtgca gtcgtctctt gctgcaggta 3120
   gccacaccct gcgcgcgcca tggcggctct ggtcacgtcc cagctcgcca cctccggcac 3180
   cgtcctcagc gtcaccgaca gattccggcg tccaggtttt cagggcctga ggccccggaa 3240
   cccggcggat gcggcgctcg gcatgaggac tgtcggagcg agcgccgccc caaagcaaag 3300
   caggaaaccg caccgattcg accggcggtg cctctccatg gtggtgcgcg ccacgggcag 3360
   cggcggcatg aacctcgtgt tcgtcggcgc cgagatggcg ccctggagca agactggcgg 3420
   cctcggcgac gtcctcgggg gcctccccgc cgccatggcc gtaagcttgc gccactgcct 3480
   tcttataaat gtttcttcct gcagccatgc ctgccgttac aacgggtgcc gtgtccgtgc 3540
   aggccaacgg tcaccgggtc atggtcatct ccccgcgcta cgaccagtac aaggacgcct 3600
   gggacaccag cgtcatctcc gaggtatata tccgccacat gaattatcac aattcacatg 3660
   ctcctgcaca tttctgcaag actttactga ctggctggat ctcgcagatc aaggtcgttg 3720
   acaggtacga gagggtgagg tacttccact gctacaagcg cggggtggac cgcgtgttcg 3780
   tcgac                                                             3785
<210> 2
   <211> 29
   <212> DNA
   <213> Artificial Sequence
<220>
   <223> Description of Artificial Sequence: Oligonucleotide
<400> 2
   cacgcaaagg cgcgtcggcc agccacgac 29
<210> 3
   <211> 21
   <212> DNA
   <213> Artificial Sequence
<220>
   <223> Description of Artificial Sequence: Oligonucleotide
<400> 3
   agaaacaaac aaacaaacaa a 21
<210> 4
   <211> 72
   <212> DNA
   <213> Artificial Sequence
<220>
   <223> Description of Artificial Sequence: Oligonucleotide
<400> 4
   cctttcagga cgatgcttcg gtgccttaag acacctacct ttgtgtctat gacatgtgag 60
   cccaacagtg gc                                                     72
<210> 5
   <211> 26
   <212> DNA
   <213> Artificial Sequence
<220>
   <223> Description of Artificial Sequence: Oligonucleotide
<400> 5
   cccgtctagg cgttcggtgt ccggcc 26
<210> 6
   <211> 12
   <212> DNA
   <213> Artificial Sequence
<220>
   <223> Description of Artificial Sequence: Oligonucleotide
<400> 6
   caggagcctc ga 12
<210> 7
   <211> 24
   <212> DNA
   <213> Artificial Sequence
<220>
   <223> Description of Artificial Sequence: Oligonucleotide
<400> 7
   tcagccagtt ccaccccgtg cacg 24
<210> 8
   <211> 18
   <212> DNA
   <213> Artificial Sequence
<220>
   <223> Description of Artificial Sequence: Oligonucleotide
<400> 8
   atactctggt catgttaa 18
<210> 9
   <211> 20
   <212> DNA
   <213> Artificial Sequence
<220>
   <223> Description of Artificial Sequence: Oligonucleotide
<400> 9
   atggcggctc tggtcacgtc 20
<210> 10
   <211> 20
   <212> DNA
   <213> Artificial Sequence
<220>
   <223> Description of Artificial Sequence: Oligonucleotide
<400> 10
   aggccgccag tcttgctcca 20

## Beschreibung

Die vorliegende Erfindung betrifft Promotoren, die eine karyopsenspezifische Expression oder Suppression von Genen in genetisch modifizierten Pflanzen erlauben, Verfahren zur gewebespezifischen Genexpression oder Gensuppression in Pflanzen, Expressionskassetten, rekombinante Vektoren und Wirtszellen, die solche Promotoren enthalten, mit besagten Promotoren transformierte transgene Pflanzenzellen und Pflanzen, sowie Verfahren zur Herstellung solcher Pflanzenzellen und Pflanzen.

Nachfolgend werden Dokumente aus dem Stand der Technik zitiert, deren Offenbarungsgehalt hiermit durch Referenz Teil dieser Anmeldung ist.

Der Einsatz von Pflanzen, die mit Hilfe gentechnischer Verfahren in ihrem Erbgut verändert wurden, hat sich in vielen Bereichen der Landwirtschaft als vorteilhaft erwiesen, um bestimmte Eigenschaften auf Nutzpflanzen zu übertragen. Die vornehmlichen Ziele sind vor allem Pflanzenschutz und zum anderen eine Qualitäts- und Ertragssteigerung der erntbaren Produkte.

Zahlreiche Verfahren zur genetischen Modifikation dikotyler und monokotyler Pflanzen sind bekannt (vgl. u.a. Gasser und Fraley, Science 244 (1989), 1293-1299; Potrykus, Ann. Rev. Plant Mol. Biol. Plant Physiol. 42 (1991), 205-225). Oft basieren diese auf der Übertragung von Genkonstrukten, die in den meisten Fällen Kombinationen von bestimmten codierenden Regionen von Strukturgenen mit Promotorregionen derselben oder anderer Strukturgene, sowie Transkriptionsterminatoren darstellen.

Die Bereitstellung von Promotoren ist im Zusammenhang mit der Expression von Strukturgenen von großer Bedeutung für die Herstellung von transgenen Pflanzen, da die Spezifität eines Promotors ausschlaggebend dafür ist, zu welchem Zeitpunkt, in welchen Gewebetypen unter welchen physiologischen Bedingungen und mit welcher Intensität ein transferiertes Gen in der modifizierten Pflanze exprimiert wird.

Die Initiation und Regulation der Transkription unterliegt dem als Promotor bezeichneten DNA-Abschnitt eines Gens. In der Regel liegen Promotorsequenzen im 5'-flankierenden Bereich eines transkribierten Gens. Einzelne Elemente eines Promotors (z.B. transkriptionelle Enhancer) können unter Umständen auch im 3'-flankierenden Bereich oder innerhalb von Intron-Sequenzen eines Gens lokalisiert sein (Kuhlemeier (1992) Plant Mol. Biol. 19: 1-14; Luehrsen (1994) The Maize Handbook, 636-638).

Eine Vielzahl von Promotoren, die die Expression von transferierten Genen oder Strukturgenen in Pflanzen steuern können, ist bereits bekannt. Der am häufigsten verwendete Promotor ist der 35S CaMV-Promotor (Franck et al., Cell 1 (1980), 285-294), der zu einer konstitutiven Expression des eingeführten Gens führt.
Häufig werden auch induzierbare Promotoren eingesetzt, beispielsweise zur Wundinduktion (DE-A-3843628), chemischen Induktion (Ward et al., Plant Molec. Biol. 22 (1993), 361-366) oder Lichtinduktion (Fluhr et al., Science 232 (1986), 1106-1112).

Auch die Verwendung zell- und gewebespezifischer Promotoren wurde beschrieben: schließzellenspezifische (DE-A-4207358), samen-, knollen- und fruchtspezifische (zusammengefaßt in Edwards and Coruzzi, Annu. Rev. Genet. 24 (1990), 275-303; DE-A-3843627), phloemspezifische (Schmülling et al., Plant Cell 1 (1989), 665-670), wurzelknöllchenspezifische (DE-A-3702497) oder meristemspezifische Genexpression (Ito et al., Plant Mol. Biol. 24 (1994), 863-878).

Die Verwendung der beschriebenen Promotoren ist oft mit Nachteilen verbunden. Promotoren, die eine konstitutive Expression der von ihnen kontrollierten Gene bewirken, können beispielsweise zur Erzeugung herbizidtoleranter und pathogenresistenter Pflanzen eingesetzt werden, haben aber den Nachteil, daß die Produkte der von ihnen kontrollierten Gene in allen Teilen der Pflanze vorliegen, was unerwünscht sein kann, z.B. wenn die Pflanzen der Ernährung dienen sollen. Ein negativer Aspekt der gewebe- und/oder entwicklungsunabhängigen Expression eines Transgens kann außerdem in einer unerwünschten Wirkung auf die Pflanzenentwicklung bestehen. Induzierbare Promotoren sind gleichfalls mit Nachteilen verbunden, da die Induktionsbedingungen bei landwirtschaftlich genutzten Pflanzen im Freiland typischerweise schwer kontrollierbar sind.

Darüberhinaus ist es zur Bewältigung verschiedener Ansätze der genetischen Veränderung von Pflanzen erforderlich, Gene, die unterschiedlich reguliert werden sollen, unter die Kontrolle verschiedener Promotoren zu stellen. Es ist daher notwendig, verschiedene Promotorsysteme mit unterschiedlicher Spezifität zur Verfügung zu stellen.

Die kontrollierte Expression von Transgenen ist zum Beispiel für das Einbringen von Resistenzeigenschaften oder die Modifikation von Stoffwechselvorgängen in Pflanzen von großem Nutzen. Soll ein Transgen in definierte Stoffwechselwege einer Pflanze eingreifen, z.B. einen neuen Inhaltstoff produzieren oder vor Pathogenbefall schützen, ist seine räumlich und/oder zeitlich kontrollierte Expression nur unter Verwendung eines induzierbaren und/oder gewebe- und/oder entwicklungsspezifischen Promotors möglich. Erst dadurch wird die gezielte Produktion von erwünschten Inhaltsstoffen in einem definierten Entwicklungsstadium oder Gewebe der Pflanze ermöglicht. Z. B. kann für die Anwendung der Antisense-Technologie, in der die Expression von pflanzeneigenen Genen verhindert werden soll, der Einsatz von gewebe- und/oder entwicklungsspezifischen Promotoren gegenüber einer gewebe- und/oder entwicklungsunabhängigen Expression von Vorteil sein: Der Antisense-Effekt tritt so genau in dem Entwicklungsstadium bzw. Gewebe der Pflanze auf, in dem auch das pflanzeneigene Gen exprimiert wird.

Promotoren, die die Genexpression in der Karyopse regulieren sind bisher nur in begrenzter Zahl bekannt. Zur Bewältigung bestimmter Ansätze der genetischen Veränderung von Pflanzen ist es erforderlich, alternative Promotorsysteme zur Genexpression in der Karyopse zur Verfügung zu stellen, die im Vergleich zu den bekannten Systemen unterschiedlich reguliert sind.

Aus verschiedenen Pflanzenspezies wurden Gene der Stärkebiosynthese isoliert, deren Genprodukte spezifisch im Speichergewebe der Karyopse, nicht aber in vegetativen Geweben exprimiert werden, z.B. entsprechende Gene bzw. cDNA-Klone der GBSS I. Dazu gehört der *waxy* Locus aus Mais (Klösgen et al. (1986) Mol. Gen. Genet. 203: 237-244), sowie aus Gerste (Rohde et al. (1988) Nucleic Acid Research 16, No. 14: 7185-7186), Reis (Wang et al. (1990) Nucleic Acid Research 18: 5898), Kartoffel (van der Leij et al. (1991) Mol. Gen. Genet. 228: 240-248), Erbse (Dry et al. (1992) Plant J. 2: 193-202), Maniok (Salehuzzaman et al. (1993) Plant Mol. Biol. 20: 947-962), Hirse (Hsingh et al. (1995) Acc.Nr. U23954) und Zuckerrübe (Schneider et al. (1999) Mol. Gen. Genet. 262: 515-524).

Auch aus Weizen wurde bereits eine *waxy*-cDNA isoliert und sequenziert (Clark et al. (1991) Plant Mol. Biol. 16: 1099-1101; Ainsworth et al. (1993) Plant Mol. Biol. 22: 67-82). Ein weiterer Klon der GBSS I wurde aus einer cDNA-Bank von ca. 20 Tage alten Weizenkaryopsen isoliert (Block (1997) "Isolierung, Charakterisierung und Expressionsanalysen von Stärkesynthase-Genen aus Weizen" (Triticum aestivum L.), Dissertation, Universität Hamburg). Von dieser GBSS I konnte nachgewiesen werden, daß sie in Karyopse und Pollen exprimiert wird.

Während inzwischen drei homologe *waxy* Strukturgene, die auf den Chromosomen 7A, 4A und 7D des hexaploiden Weizes liegen, isoliert wurden (Murai et al. (1999) Gene 234: 71-79), sind die Promotorsequenzen dieser oder anderer genomischer Klone aus Weizen bisher unbekannt. Bekannt sind lediglich die 5'-flankierenden Bereiche der GBSS I aus Gerste (GenBank Acc.No. X07931), Löwenmäulchen (GenBank Acc.No. AJ006294), Reis (GenBank Acc.No. AB008794, AB008795), Kartoffel (GenBank Acc.No. X58453) und Mais (GenBank Acc.No. X03935).

Ein cDNA-Klon einer stärkekorngebundenen Stärkesynthase vom Typ II (GBSS II), die nicht im Endosperm, sondern nur in Blättern und im Perikarp von Weizen exprimiert wird, konnte kürzlich isoliert werden (Vrinten & Nakamura (2000) Plant Physiol.122: 255-263). In diploidem Weizen (*Triticum monococcum* L.) wurde außerdem auf Proteinebene eine 56kDa große Isoform einer GBSS beschrieben (Fujita & Taira (1998) Planta 207: 125-132). Diese Isoform kann im Perikarp, Aleuron und Embryo von unreifen Karyopsen nachgewiesen werden.

Der vorliegenden Erfindung liegt somit die Aufgabe zugrunde, Mittel bereitzustellen, die eine gezielte karyopsenspezifische Genexpression in genetisch modifizierten Pflanzen ermöglichen, vorzugsweise in monokotylen Pflanzen.

Durch den Einsatz der erfindungsgemäßen Mittel, d.h. der erfindungsgemäßen Nukleinsäuremoleküle, Vektoren, Zellen oder Pflanzen, wird ein gewebe- und/oder entwicklungsspezifisch definierter Eingriff in den pflanzlichen Stoffwechsel ermöglicht, z.B. in die Biosynthese von Speicherstärke oder die Nutzung der Karyopse als Speicher- oder Syntheseorgan für Stärke oder andere Reservestoffe (z.B. Polyglukane, Fettsäuren, ggf. modifizierte Speicherproteine oder biopolymere Kunststoffe) ermöglicht.

Unter der Kontrolle der erfindungsgemäßen Promotorsequenzen können somit Gene, insbesondere während der Kornentwicklung von Getreiden, spezifisch und zu einem frühen Zeitpunkt in der Karyopse exprimiert werden.

Mittels der erfindungsgemäßen Promotorsequenzen können darüber hinaus Gene, insbesondere während der Kornentwicklung von Getreiden, spezifisch und zu einem frühen Zeitpunkt in der Karyopse durch sog., gene silencing'-Strategien (Cosuppression) supprimiert werden. Cosuppressionsstrategien unter Einsatz von Promotoren sind ausführlich von Vaucheret et al. (Vaucheret et al., 1998, 16(6), 651-659) beschrieben worden. Insbesondere der Abschnitt ,Transcriptional trans-inactivation'auf Seite 652 des Artikels von Vaucheret et al. sei hiermit durch Referenz Teil dieser Anmeldung, der speziell Cosuppressionsstrategien beschreibt, für die die erfindungsgemäßen Promotoren geeignet sind, insbesondere solche, die dort als 'ectopic trans-inactivation' bezeichnet werden können (Matzke et al., 1994, Mol Gen Genet. 244, 219-229). Solchermaßen können die erfindungsgemäßen Promotoren dazu verwendet werden, die Genexpression beliebiger Gene zu supprimieren, die unter der Kontrolle eines Promotors stehen, der als Ziel für die Cosuppression durch die erfindungsgemäßen Promotoren zugänglich ist. Gegebenenfalls ist hierfür bereits ein Sequenzabschnitt von nur etwa 90 bp Länge ausreichend.

Die erfindungsgemäßen Promotoren ermöglichen so beispielsweise die gezielte Veränderung von Speicherstärke: Um eine möglichst vielfältige Anwendung von Stärke für die unterschiedlichsten industriellen Bedürfnisse zu ermöglichen, ist es wünschenswert Pflanzen bereitzustellen, die Stärken mit definierten Eigenschaften synthetisieren können. So werden z.B. für die verarbeitende Industrie entscheidende Eigenschaften wie Löslichkeit, Verkleisterungsverhalten, Retrogradierungstendenz, Viskosität und Komplexiervermögen durch das Verhältnis von Amylose und Amylopektin zueinander, dem Verzweigungsgrad des Amylopektins und die Derivatisierung der Polymere bestimmt. Eine gezielte Modifizierung solcher Eigenschaften ersetzt aufwendige Verfahren zur Trennung von Amylose und Amylopektin oder die kostspielige chemische Modifizierung von Stärke.

Eine begrenzte Möglichkeit, solche Pflanzen zu erhalten, besteht in der Anwendung klassischer Züchtungsmethoden. Durch Kreuzung spontan auftretender Mutanten gelang so zum Beispiel die Herstellung eines (amylosefreien) *"waxy"* Weizens (Nakamura et al. (1995) Mol. Gen. Genet. 248: 253-259). Aufgrund des polyploiden Charakters des kommerziell bedeutenden Brot-Weizens sind Mutationen welche die Stärkestruktur betreffen nicht leicht zu erkennen, da sie von intakten Allelen kompensiert werden. Die Anwendung klassischer Züchtungsmethoden erweist sich daher als schwierig. Außerdem kann nur auf bereits vorhandene Enzymaktivitäten zurückgegriffen werden. Neue Aktivitäten, die bisher nicht in Pflanzen identifiziert wurden oder die in Pflanzen (oder anderen Organismen) identifiziert wurden, die nicht mit der Zielpflanze kreuzbar sind, können ebenfalls nicht mit Hilfe züchterischer Verfahren bearbeitet werden.

Eine Alternative besteht in der gezielten Modifikation stärkeproduzierender Pflanzen durch gentechnische Methoden. Voraussetzung hierfür ist jedoch neben der Identifizierung und Isolierung von Genen, deren Genprodukte an der Stärkesynthese und/oder Stärkemodifikation beteiligt sind, der Einsatz spezifischer Promotoren, die eine gewebe- und/oder entwicklungsspezifische Expression der von ihnen kontrollierten Gene in den stärkebildenden Geweben vermitteln.

Durch den Einsatz der erfindungsgemäßen Promotorsequenzen können außerdem auch solche Gene eingebracht werden, die dem Getreideendosperm eine modifizierte Funktion als Speichergewebe für andere Speicherstoffe verleihen.

Diese Aufgaben werden erfindungsgemäß durch die Bereitstellung der in den Patentansprüchen charakterisierten Ausführungsformen gelöst.

Es wurde nun gefunden, daß überraschenderweise ein Promotor wie nachfolgend definiert in Pflanzen eine karyopsenspezifische Expression einer von ihm kontrollierten codierenden Nucleotidsequenz bewirkt.

Somit betrifft die vorliegende Erfindung ein Nucleinsäuremolekül mit der Funktion eines karyopsenspezifischen Promotors, das
a) die durch Seq ID No. 1 definierte oder durch DSM 13398 (Plasmid p 11/1) hinterlegte Nucleinsäuresequenz umfaßt;
b) ein oder mehrere Sequenzelemente umfaßt, ausgewählt aus der Gruppe bestehend aus
   i) cacgcaaagg cgcgtcggcc agccacgac (Seq ID No. 2);
   ii) agaaacaaac aaacaaacaa aaaagt (Seq ID No. 3);
   iii) cctttcagga cgatgcttcg gtgccttaag acacctacc tttgtgtcta tgacatgtga gcccaacag atggct (Seq ID No. 4);
   iv) cccgtctagg cgttcggtgt ccggcc (Seq ID No. 5);
   v) cagggagcct tcga (Seq ID No. 6);
   vi) tcagccagtt ccaccccgtg cacg (Seq ID No. 7) und
   vii) tactctggtc atgttaa (Seq ID No. 8);
c) einen funktionalen Teil der in a) genannten Nucleinsäuresequenz umfaßt; wobei der funktionale Teil die Sequenzabschnitte 948-3139;1006-3139; 1240-3139; 1259-3139; 1382-3139; 1486-3139;1514-3139; 1655-3139; 1822-3139; 1887-3139; 2138-3139 oder 2176-3139 der Seq. ID No.1 umfasst; oder
d) eine Sequenz umfaßt, die mit mindestens einer der in a) genannten Nucleinsäuresequenzen zu etwa 90-99 % identisch ist.

Im Rahmen der vorliegenden Erfindung werden die Begriffe "erfindungsgemäßes Nukleinsäuremolekül" und "erfindungsgemäßer Promotor" im allgemeinen als Synonyme verwendet.

In einer bevorzugten Ausführungsform sind die erfindungsgemäßen Promotoren solche von pflanzlichen Genen, vorzugsweise von monokotylen Pflanzen, oder von solchen abgeleitet. In einer weiteren, bevorzugten Ausführungsform sind die erfindungsgemäßen Promotoren zur Expression oder Suppression von Genen in genetisch modifizierten Pflanzen geeignet, vorzugsweise in monokotylen Pflanzen, insbesondere Expression oder Suppression von Stärkesynthase-Genen. Die erfindungsgemäßen Promotoren können hierbei aus pflanzlichen Genen stammen, durch rekombinante DNA-Techniken modifiziert sein und/oder synthetisch hergestellt werden.

Die erfindungsgemäßen Promotoren können z.B. modifiziert werden, indem sie mit weiteren *cis*-regulatorischen Elementen kombiniert werden. So können die erfindungsgemäßen Promotoren zusätzlich mit Enhancer-Elementen kombiniert werden, um die Expression des korrespondierenden Nucleinsäuremoleküls zu verstärken, ohne jedoch seine gewebespezifische Expression zu beeinflussen. Auch individuelle cis-Elemente (s.u.) der isolierten Promotoren können ebenfalls zu regulatorischen Einheiten miteinander kombiniert werden.

Im Zusammenhang mit der vorliegenden Erfindung wird unter einem "Promotor" eine DNA-Sequenz verstanden, die den regulatorischen Anteil eines Gens, vorzugsweise eines Strukturgens, umfaßt. Unter dem "regulatorischen Anteil" eines Gens wird derjenige Anteil verstanden, der die Expressionsbedingungen des Gens bestimmt. Ein regulatorischer Anteil besitzt ein Sequenzmotiv, mit dem Transkriptionsfaktoren und RNA-Polymerase in Wechselwirkung treten und die Transkription des codierenden Anteils des Gens einleiten. Darüber hinaus kann der regulatorische Anteil ein oder mehrere positive regulatorische Elemente, sogenannte Enhancer umfassen. Er kann zusätzlich oder an deren Stelle aber auch negativ regulatorische Elemente, sogenannte Silencer enthalten. Unter einem "Strukturgen" wird im allgemeinen eine genetische Einheit aus regulatorischem und codierendem Anteil verstanden, deren Genprodukt im allgemeinen ein Protein ist. Die Information für die primäre Aminosäuresequenz des Genprodukts ist im codierenden Anteil des Strukturgens enthalten, während der regulatorische Anteil bestimmt, wann, in welchen Geweben, unter welchen physiologischen Bedingungen und in welchen Mengen das Transkript des codierenden Anteils gebildet wird, nach dessen Vorlage das Genprodukt synthetisiert wird.

Unter dem Begriff "karyopsenspezifisch" versteht man im Rahmen der vorliegenden Erfindung, daß ein unter der Kontrolle eines erfindungsgemäßen Promotors stehendes Gen in der Karyopse, d.h. Endosperm, Perikarp und Scutellum und/oder Pollen exprimiert wird, vorzugsweise zu einem frühen Zeitpunkt nach der Befruchtung, d.h. etwa 15-5dap (dap = Tage nach der Bestäubung), vorzugsweise etwa 10-5 dap, insbesondere etwa 5 dap. Insbesondere ist Karyopsenspezifität im Rahmen der vorliegenden Erfindung dann gegeben, wenn der erfindungsgemäße Promoter die Expression eines Gens in der Karyopse im Vergleich zu anderen Geweben wie z.B. maturen Blättern oder Wurzeln begünstigt und in der Karyopse eine signifikante Erhöhung, d.h. eine 2- bis 5-fach, vorzugsweise 5- bis 10-fach, insbesondere 10- bis 100-fach höhere Expressionsrate bewirkt.

Im Zusammenhang mit der vorliegenden Erfindung kann Karyopsenspezifität z.B. durch übliche Reportergen-Experimente analysiert werden. Zur Testung einer isolierten Promotorsequenz auf deren Promotoraktivität in der Karyopse kann der Promotor beispielsweise in einer Expressionskassette bzw. in einen Vektor zur Pflanzentransformation operativ mit einem Reportergen, wie z.B. dem β-Glucuronidasegen (gus) aus *E. coli,* verknüpft werden. Dieses Konstrukt wird dann zur Transformation von Pflanzen verwendet. Anschließend wird die Expression der β-Glucuronidase (GUS) in der Karyopse im Vergleich zu anderen Geweben, wie z.B. maturen Blättern oder Wurzeln bestimmt, wie z.B. bei Martin et al. (The GUS Reporter System as a Tool to Study Plant Gene Expression, In: GUS Protocols: Using the GUS Gene as a Reporter of Gene Expression, Academic Press (1992), 23-43) beschrieben.

Der Begriff "Karyopse" ist dem Fachmann geläufig und umfaßt insbesondere Perikarp und Endosperm. Da diese Gewebe eine dynamische Entwicklung durchlaufen, korreliert z.B. die Entwicklung des Endosperms in verschiedene Zell- oder Gewebetypen mit unterschiedlichen biochemischen Aktivitäten, bedingt durch eine differenzielle Genexpression. Ergänzend sei auf Olsen et al. verwiesen (Olsen et al., 1999, Trends in Plant Science 4 (7), 253-257).

Der erfindungsgemäße Promotor erlaubt eine karyopsenspezifische Genexpression einer von ihm kontrollierten codierenden Nucleotidsequenz. Er stellt eine interessante Alternative zu bekannten Promotoren dar, weil er auch die Genexpression im Perikarp vermitteln kann und darüber hinaus bereits zu einem sehr frühen Zeitpunkt in der Karyopse aktiv ist, d.h. etwa 15-5 dap, vorzugsweise etwa 10-5 dap, insbesondere etwa bei 5 dap. Mit Hilfe des erfindungsgemäßen Promotors kann insbesondere die Expression von solchen Genen effektiv gesteuert werden, deren Genprodukte am Stärkemetabolismus in monokotylen Pflanzen und insbesondere Weizen beteiligt sind.

Vielfältige Verwendungsmöglichkeiten stehen für die erfindungsgemäßen Promotoren zur Verfügung. Beispielsweise wird die Herstellung transgener Pflanzen ermöglicht, die aufgrund eines modifizierten Metabolismus in der Karyopse eine qualitativ und/oder quantitativ veränderte Speicherstoffzusammensetzung in ihrem Speichergewebe, d.h. im Getreidekorn aufweisen.

Neben einem Promotor, der die gesamte durch SEQ ID No. 1 definierte Sequenz, bzw. die entsprechend durch DSM 13398 hinterlegte Sequenz aufweist, betrifft die vorliegende Erfindung auch Promotoren, die einen funktionalen Teil dieser Sequenz aufweisen und die in Pflanzen eine karyopsenspezifische Expression einer von ihnen kontrollierten codierenden Nucleotidsequenz bewirken.

Ein Maß für die Promotoraktivität ist beispielsweise die für ein bestimmtes Markergen ermittelte Expressionsrate, wenn es unter der regulativen Kontrolle des erfindungsgemäßen Promotors steht. Beispiele für geeignete Markergene sind das β-Glucuronidase-Gen (gus)aus *E*. *coli* (Jefferson (1987) Plant Molecular Biology Reporter Vol.5 (4): 387-405) oder das Green-Fluorescence-Protein-Gen (gfp) (Baulcombe et al., Plant J. 7 (16) (1993), 1045-1053). Die Organ- bzw. Gewebespezifität läßt sich leicht durch Vergleich der an einzelnen Geweben bzw. Organen der Pflanze ermittelten Expressionsraten für besagte Markergene bestimmen. Funktionale Teile der Promotorsequenzen umfassen im Rahmen der vorliegenden Erfindung sowohl natürlich vorkommende Varianten der erfindungsgemäßen Sequenzen, als auch künstliche, z.B. durch chemische Synthese erhaltene Nucleotidsequenzen.

Funktionale Teile der erfindungsgemäßen Promotorsequenz umfassen dabei auch solche Promotorvarianten, deren Promotoraktivität, verglichen mit dem unmodifizierten Promotor (Wildtyp), abgeschwächt oder verstärkt ist.

Insbesondere werden unter funktionalen Teilen der erfindungsgemäßen Promotorsequenzen die durch Deletionsanalyse (vgl. Beispielteil) identifizierbaren Bereiche verstanden, vorzugsweise die Sequenzabschnitte 948-3139; 1006-3139; 1240-3139; 1259-3139; 1382-3139; 1486-3139; 1514-3139; 1655-3139; 1822-3139; 1887-3139; 2138-3139 und 2176-3139 der Seq ID No. 1.

Prinzipiell wird die Aktivität eines eukaryontischen RNA-Polymerase II-Promotors durch das synergistische Zusammenwirken verschiedener *trans*-aktiver Faktoren (DNA-bindende Moleküle wie Proteine oder Hormone) bedingt, welche an die verschiedenen im Promotor vorhandenen *cis*-regulatorischen DNA-Elemente, in der Regel etwa 10-20 Nukleotide lange DNA-Bereiche binden. Diese Faktoren wechselwirken direkt oder indirekt mit einem oder mehreren Faktoren der basalen Transkriptionsmaschinerie, was letztlich zur Ausbildung eines Präinitiationskomplexes in der Nähe der Transkriptionsstartstelle führt (Drapkin et al., Current Opinion in Cell Biology 5 (1993), 469-476). Man kann von einem modularen Aufbau eukaryontischer RNA-Polymerase II-Promotoren ausgehen, wobei die cis-Elemente (Module) als Teilkomponenten des Promotors dessen Aktivität im einzelnen determinieren (Tjian und Maniatis, Cell 77 (1994), 5-8).

Einzelne, potentiell Gewebespezifität vermittelnde Subdomänen des erfindungsgemäßen Promotors können beispielsweise durch Fusion mit einer Minimalpromotor-Reportergen-Kassette identifiziert werden. Unter einem Minimalpromotor versteht man eine DNA-Sequenz, die eine TATA- Box, die sich etwa 20 bis 30 Basenpaare stromaufwärts von der Transkriptionsstarstelle befindet, oder eine Initiatorsequenz (Smale und Baltimore, Cell 57 (1989), 103-113; Zawel und Reinberg, Proc. Natl. Acad. Sci. 44 (1993), 67-108; Conaway und Conaway, Annu. Rev. Biochem 62 (1993), 161-190) umfaßt. Beispiele für Minimalpromotoren sind der -63 bis +8 Δ35S-Promotor (Frohberg, Dissertation an der FU Berlin FB Biologie (1994)), der -332 bis +14 Minimal-Patatin-Classl-Promotor sowie der -176 bis +4-Minimal-PetE-Promotor (Pwee et al., Plant J. 3 (1993), 437-449.)

Desweiteren können Subdomänen bzw. *cis-*Elemente des erfindungsgemäßen Promotors auch über Deletionsanalysen bzw. Mutagenesen identifiziert werden (Kawagoe et al., Plant J. 5(6) (1994), 885-890). Der Test auf Funktionalität einer solchen Subdomäne oder cis-Elements des Promotors kann *in planta* durch den Nachweis von Reportergenaktivität in stabil transformierten Zellen erfolgen.

In einer weiteren Ausführungsform betrifft die vorliegende Erfindung daher insbesondere durch Di- oder Multimerisierung von Subdomänen bzw. cis-Elementen von SEQ ID No.1 erhaltenen Modifikationen von Seq. ID No. 1.

In einer weiteren Ausführungsform der Erfindung wird die Erhöhung der Promotoraktivität im Vergleich zum Wildtyp durch die Kombination des erfindungsgemäßen Promotors mit einem sogenannten Enhancer erreicht.

In der Literatur sind verschiedene Enhancer beschrieben worden, die in der Regel eine gewebespezifische Erhöhung der Expression bewirken, wobei die Gewebespezifität im allgemeinen durch den jeweils verwendeten Enhancer determiniert wird (Benfey et al., Science 250 (1990), 959-966; Benfey et al., EMBO J. 8 (1989), 2195-2202; Chen et al., EMBO J. 7, (1988), 297-302; Simpson et al., Nature 323 (1986), 551-554).

Darüberhinaus gibt es auch Enhancer, wie z.B. den PetE Enhancer (Sandhu et al., Plant Mol. Biol. 37 (1998), 885-896), die nicht gewebespezifisch wirken und somit als quantitative Verstärkerelemente vor den erfindungsgemäßen Promotor gesetzt werden können, um die Expression in der Karyopse zu erhöhen, ohne die Qualität der Gewebespezifität des erfindungsgemäßen Promotors zu verändern.

Ferner können auch synthetische Enhancer verwendet werden, die beispielsweise von natürlich vorkommenden Enhancern abgeleitet sind und/oder durch Kombination verschiedener Enhancer erhalten werden.

Ebenso betrifft die vorliegende Erfindung auch Promotoren, die eine Nucleotidsequenz aufweisen, die mit der durch SEQ ID No. 1 definierten bzw. durch DSM 13398 hinterlegten Nucleotidsequenz hybridisiert, vorzugsweise unter stringenten Bedingungen und die in Pflanzen einen karyopsenspezifischen Einfluß auf die Expression einer von ihnen kontrollierten codierenden Nucleotidsequenz ausüben.

Der Ausdruck "stringente Bedingungen" bedeutet dabei beispielsweise Hybridisierungsbedigungen, wie sie in Sambrook et al. (Molecular Cloning, A Laboratory Manual, 2. Aufl. (1989), Cold Spring Harbor Laboratory Press, Cold Spring Harbor, NY) beschreiben sind. Insbesondere findet eine stringente Hybridisierung unter den folgenden Bedingungen statt:
Hybridisierungspuffer: 2x SSC; 10x Denhardt's Lösung (Fikoll 400 + PEG + BSA; Verhältnis 1:1:1); 0.1 % SDS; 5 mM EDTA; 50 mM Na₂HPO₄; 250 µg/ml
Heringsperma-DNA; 50 µg/ml tRNA; oder 0.25 M Natriumphosphatpuffer pH 7.2, 1 mM EDTA, 7% SDS
Hybridisierungstemperatur T= 65 bis 68 °C;
Waschpuffer 0.2 x SSC; 0.1 % SDS;
Waschtemperatur T = 65 bis 68° C.

Vorzugsweise weisen derartige Promotoren eine Sequenzidentität von mindestens 30%, bevorzugt von mindestens 40%, bevorzugt von mindestens 50%, besonders bevorzugt mindestens 60%, insbesondere bevorzugt von mindestens 70% und vorteilhafterweise von mindestens 80%, vorzugsweise mindestens 90% und insbesondere bevorzugt mindestens 95% zu der unter Seq ID No. 1 gezeigten Promotorsequenz oder Teilen davon auf. Vorzugsweise wird die Sequenzidentität derartiger Promotorsequenzen durch Vergleich mit der unter SEQ ID No. 1 dargestellten Nucleotidsequenz bestimmt. Wenn zwei zu vergleichende Sequenzen eine unterschiedliche Länge aufweisen, bezieht sich die Sequenzidentität vorzugsweise auf den prozentualen Anteil der Nucleotidreste der kürzeren Sequenz, die identisch sind mit den Nucleotidresten der längeren Sequenz. Die Sequenzidentität kann z. B. durch Verwendung von Computerprogrammen wie das Bestfit-Programm (Wisconsin Sequence Analysis Package, Version 8 for Unix, Genetics Computer Group, University Research Park, 575 Science Drive Madison, WI 53711) bestimmt werden. Bestfit nützt den lokalen Homologiealgorithmus von Smith und Waterman, Advances in Applied Mathematics 2 (1981), 482-489, um das Segment mit höchster Sequenzidentität zwischen zwei Sequenzen zu finden. Bei der Anwendung von Bestfit oder einem anderen Sequenz-Alignment-Programm zur Bestimmung, ob eine bestimmte Sequenz beispielsweise zu 95% identisch ist mit einer Referenzsequenz der vorliegenden Erfindung, werden die Parameter vorzugsweise so eingestellt, daß der Prozentanteil der Identität über die gesamte Länge der Referenzesequenz berechnet wird und daß Homologielücken ("gaps") von bis zu 5% der Gesamtzahl der Nucleotide in der Referenzsequenz erlaubt sind. Bei der Verwendung von Bestfit können die sogenannten optionalen Parameter bei ihren voreingestellten ("default") Werten belassen werden. Die Abweichungen, die bei dem Vergleich einer gegebenen Sequenz mit den oben beschriebenen Sequenzen der Erfindung auftreten, können beispielsweise durch Addition, Deletion, Substitution, Insertion oder Rekombination verursacht sein. Promotorsequenzen, die wie oben beschrieben mit der durch SEQ ID No. 1 definierten bzw. durch DSM 13398 hinterlegten Nucleotidsequenz hybridisieren, stammen vorzugsweise aus pflanzlichen Organismen, bevorzugt aus höheren Pflanzen, besonders bevorzugt aus monokotylen Pflanzen, insbesondere bevorzugt aus Gramineen und ganz besonders Pflanzen der Gattung *Triticum.*

Ferner betrifft die vorliegende Erfindung auch Promotoren, die einen funktionalen Teil der erfindungsgemäßen Promotoren aufweisen und die in Pflanzen eine karyopsenspezifische Expression einer von ihnen kontrollierten codierenden Nucleotidsequenz bewirken und die ein oder mehrere Sequenzen der Seq ID No. 2-Seq ID No. 8 umfassen.

In einer besonders bevorzugten Ausführungsform der Erfindung weist der erfindungsgemäße Promotor die gesamte Seq ID No. 1 oder einen funktionalen Teil der durch SEQ ID No. 1 definierten bzw. durch DSM 13398 hinterlegten Nucleotidsequenz auf, insbesondere die Nukleotide 948-3139; 1006-3139; 1240-3139; 1259-3139; 1382-3139; 1486-3139; 1514-3139; 1655-3139; 1822-3139; 1887-3139; 2138-3139 und 2176-3139 aus der Seq ID No. 1.

Die vorliegende Erfindung betrifft weiterhin Expressionskassetten enthaltend einen oder mehrere der erfindungsgemäßen Promotoren. Unter dem Begriff "Expressionskassette" wird dabei die Kombination eines erfindungsgemäßen Promotors mit einer zu exprimierenden Nucleinsäuresequenz verstanden. Diese Nucleinsäuresequenz kann beispielsweise eine ein Polypeptid codierende Sequenz sein, z.B. ein Gen, das in sense- oder in antisense-Orientierung mit dem Promotor verknüpft sein kann. Die Nucleinsäuresequenz kann auch eine nicht-translatierbare RNA, beispielsweise eine antisense-RNA oder ein Ribozym, codieren. Diese Nucleinsäuresequenzen können in Verbindung mit dem erfindungsgemäßen Promotor benutzt werden, um Pflanzen mit verändertem Phänotyp herzustellen.

Die erfindungsgemäßen Expressionskassetten können weiterhin eine Transkriptionsterminationssequenz stromabwärts des 3'-Endes der mit dem Promotor verknüpften Nucleinsäuresequenz enthalten. Unter einer "Transkriptionsterminationssequenz" wird dabei eine DNA-Sequenz verstanden, die am 3'-Ende eines codierenden Genabschnitts lokalisiert ist und in der Lage ist, die Beendigung der Transkription und gegebenenfalls die Synthese eines Poly-A-Schwanzes hervorzurufen. Ein Beispiel für eine solche Terminationssequenz ist die des Octopinsynthasegens. Weitere sind dem Fachmann gut bekannt.

Außerdem betrifft die vorliegende Erfindung Vektoren, die mindestens einen erfindungsgemäßen Promotor enthalten.

In einer weiterhin bevorzugten Ausführungsform ist der erfindungsgemäße Promotor in einem derartigen Vektor verknüpft mit Restriktionsschnittstellen bzw. einem Polylinker, die eine Integration beliebiger Sequenzen stromabwärts des Promotors erlauben. Dabei wird unter einem "Polylinker" eine DNA-Sequenz verstanden, die Erkennungssequenzen von mindestens einem Restriktionsenzym, vorzugsweise von zwei oder mehr Restriktionsenzymen, enthält.

In einer besonders bevorzugten Ausführungsform enthält ein erfindungsgemäßer Vektor auch noch eine Sequenz für die Termination der Transkription, beispielsweise die des Octopinsynthasegens, stromabwärts des Promotors bzw. des Polylinkers.

Ebenso betrifft die vorliegende Erfindung Vektoren, die erfindungsgemäße Expressionskassetten enthalten. Gegebenenfalls enthalten die erfindungsgemäßen Vektoren Selektionsmarker, die geeignet sind, Zellen, die die erfindungsgemäßen Vektoren enthalten, zu identifizieren und gegebenenfalls zu selektionieren.

In einer bevorzugten Ausführungsform sind die erfindungsgemäßen Vektoren geeignet zur Transformation von pflanzlichen Zellen und besonders bevorzugt zur Integration von Fremd-DNA (z.B. Transgenen) in das pflanzliche Genom. Ein Beispiel für derartige Vektoren sind binäre Vektoren, die zum Teil kommerziell erhältlich sind.

Ferner betrifft die vorliegende Erfindung Wirtszellen, die mit einer erfindungsgemäßen Expressionskassette oder einem erfindungsgemäßen Vektor genetisch modifiziert sind, insbesondere pflanzliche Zellen oder mikrobielle Zellen, z.B. der Gattung *Agrobacterium.*

"Genetisch modifiziert" bedeutet dabei, daß die Wirtszelle einen erfindungsgemäßen Promotor, eine erfindungsgemäße Expressionskassette oder einen erfindungsgemäßen Vektor enthält, vorzugsweise stabil in das Genom der Wirtszelle integriert, und der Promotor bzw. die Expressionskassette entweder in die Wirtszelle oder in einen Vorgänger dieser Zelle als Fremd-DNA eingebracht wurde. D.h. die erfindungsgemäßen Wirtszellen können entweder selbst das unmittelbare Produkt eines Transformationsereignisses sein oder davon abstammende Zellen sein, die einen erfindungsgemäßen Promotor oder eine erfindungsgemäße Expressionskassette enthalten. Als Wirtszellen kommen sowohl prokaryontische, insbesondere bakterielle, als auch eukaryontische Zellen in Frage. Eukaryontische Zellen können beispielsweise Pilzzellen sein, insbesondere der Gattung *Saccharomyces.*

In einer weiteren Ausführungsform betrifft die Erfindung die Verwendung der erfindungsgemäßen Vektoren, erfindungsgemäßen Expressionskassetten oder erfindungsgemäßen Wirtszellen, insbesondere Wirtszellen der Gattung *Agrobacterium* zur Transformation von Pflanzen, Pflanzenzellen, -geweben oder - teilen.

In einer besonders bevorzugten Ausführungsform sind die erfindungsgemäßen Wirtszellen Pflanzenzellen, die im folgenden als "transgene Pflanzenzellen" bezeichnet werden.

Ferner betrifft die vorliegende Erfindung auch Pflanzen, die erfindungsgemäße Pflanzenzellen enthalten. Diese können grundsätzlich jeder beliebigen Pflanzenart, -gattung, -familie, -ordnung bzw. -klasse angehören, die gewerblich nutzbar sind. Es können sowohl monokotyle als auch dikotyle Pflanzen sein. Vorzugsweise sind die erfindungsgemäßen Pflanzen Nutzpflanzen, d.h. Pflanzen, die für den Menschen von agrarwirtschaftlichem, forstwirtschaftlichem und/oder gartenbauwirtschaftlichem Interesse sind. Bevorzugt sind dabei landwirtschaftliche Nutzpflanzen, insbesondere Getreidearten wie z.B. Weizen, Hafer, Gerste, Roggen, Mais, Reis oder Futter- und Weidegräser (wie z.B. Alfalfa, weißer oder roter Klee).

In einer weiteren Ausführungsform betrifft die vorliegende Erfindung auch Verfahren zur Herstellung von transgenen Pflanzenzellen und Pflanzen, dadurch gekennzeichnet, daß man Pflanzenzellen, -gewebe oder -teile oder Protoplasten mit einem erfindungsgemäßen Nukleinsäuremolekül, einem erfindungsgemäßen Vektor, einer erfindungsgemäßen Expressionskassette oder mit einer erfindungsgemäßen Wirtszelle, vorzugsweise einem Mikroorganismus transformiert, die transformierten Zellen, Gewebe, Pflanzenteile oder Protoplasten in einem Wachstumsmedium kultiviert und im Fall der Herstellung transgener Pflanzen daraus Pflanzen regeneriert.

In einer weiteren Ausführungsform betrifft die Erfindung die Verwendung eines oder mehrerer der erfindungsgemäßen Nukleinsäuremoleküle, Vektoren, Expressionskassetten oder gegebenenfalls Wirtszellen zur Herstellung transgener Wirtszellen, insbesondere transgener Pflanzenzellen und Pflanzen.

In einer weiteren Ausführungsform betrifft die Erfindung ein Verfahren zur karyopsenspezifischen Genexpression in Pflanzen, worin eines oder mehrere der erfindungsgemäßen Nukleinsäuremoleküle direkt oder mittels eines oder mehrerer der erfindungsgemäßen Vektoren, Expressionskassetten oder Wirtszellen stabil in das Genom einer Pflanzenzelle integriert wird und aus besagter Pflanzenzelle eine Pflanze regeneriert wird.

In einer weiteren Ausführungsform betrifft die Erfindung ein Verfahren zur karyopsenspezifischen Gensuppression in Pflanzen, worin eines oder mehrere der erfindungsgemäßen Nukleinsäuremoleküle direkt oder mittels eines oder mehrerer der erfindungsgemäßen Vektoren, Expressionskassetten oder Wirtszellen stabil in das Genom einer Pflanzenzelle integriert wird und aus besagter Pflanzenzelle eine Pflanze regeneriert wird, vorzugsweise mittels Cosuppression.

Die erfindungsgemäßen Pflanzen können nach dem Fachmann bekannten Verfahren hergestellt werden, z.B. durch Transformation pflanzlicher Zellen oder Gewebe und Regeneration ganzer Pflanzen aus den transformierten Zellen bzw. dem Gewebe.

Für die Einführung von DNA in eine pflanzliche Wirtszelle stehen eine Vielzahl von Techniken zur Verfügung. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, die Injektion, die Elektroporation von DNA, die Einbringung der DNA mittels des biolistischen Ansatzes sowie weitere Möglichkeiten.

Bei der Injektion und Elektroporation von DNA in Pflanzenzellen werden an sich keine speziellen Anforderungen an die verwendeten Plasmide gestellt. Es können einfache Plasmide wie z. B. pUC-Derivate verwendet werden. Sollen aber aus derartig transformierten Zellen ganze Pflanzen regeneriert werden, ist z.B. die Anwesenheit eines selektierbaren Markergens notwendig.

Je nach Einführungsmethode gewünschter Gene in die Pflanzenzelle können weitere DNA-Sequenzen erforderlich sein. Werden z. B. für die Transformation der Pflanzenzelle das Ti- oder Ri- Plasmid verwendet, so muss mindestens die rechte Begrenzung, häufig jedoch die rechte und linke Begrenzung der Ti- und Ri- Plasmid T-DNA als Flankenbereich mit den einzuführenden Genen verbunden werden.

Werden für die Transformation Agrobakterien verwendet, muss die einzuführende DNA in spezielle Plasmide cloniert werden, und zwar entweder in einen intermediären Vektor oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält ausserdem die für den Transfer der T-DNA notwendige vir-Region. Intermediäre Vektoren können nicht in Agrobakterien replizieren. Mittels eines Helferplasmids kann der intermediäre Vektor auf Agrobacterium tumefaciens übertragen werden (Konjugation). Binäre Vektoren können sowohl in E.coli als auch in Agrobakterien replizieren. Sie enthalten ein Selektionsmarker-Gen und einen Linker oder Polylinker, welche von der rechten und linken T-DNA Grenzregion eingerahmt werden. Sie können direkt in die Agrobakterien transformiert werden (Holsters et al. Mol. Gen. Genet. 163 (1978), 181-187). Das als Wirtszelle dienende Agrobakterium soll ein Plasmid, das eine vir-Region trägt, enthalten. Die vir-Region ist für den Transfer der T-DNA in die Pflanzenzelle notwendig. Zusätzliche T-DNA kann vorhanden sein. Das derartig transformierte Agrobakterium wird zur Transformation von Pflanzenzellen verwendet.

Die Verwendung von T-DNA für die Transformation von Pflanzenzellen ist intensiv untersucht und ausreichend in EP 120 516; Hoekema, In: The Binary Plant Vector System Offsetdrukkerij Kanters B.V., Alblasserdam (1985), Chapter V; Fraley et al., Crit. Rev. Plant. Sci., 4, 1-46 und An et al. EMBO J. 4 (1985), 277-287 beschrieben worden.

Für den Transfer der DNA in die Pflanzenzelle können Pflanzen- Explantate zweckmässigerweise mit Agrobacterium tumefaciens oder Agrobacterium rhizogenes cokultiviert werden. Aus dem infizierten Pflanzenmaterial (z. B. Blattstücke, Stengelsegmente, Wurzeln, aber auch Protoplasten oder Suspensionskultivierte Pflanzenzellen) können dann in einem geeigneten Medium, welches Antibiotika oder Biozide zur Selektion transformierter Zellen enthalten kann, wieder ganze Pflanzen regeneriert werden. Die so erhaltenen Pflanzen können dann auf Anwesenheit der eingeführten DNA untersucht werden. Andere Möglichkeiten der Einführung fremder DNA unter Verwendung des biolistischen Verfahrens oder durch Protoplastentransformation sind bekannt (vgl. z. B. Willmitzer, L., 1993 Transgenic plants. In: Biotechnology, A Multi-Volume Comprehensive Treatise (H.J. Rehm, G. Reed, A. Pühler, P. Stadler, eds.), Vol. 2, 627-659, VCH Weinheim-New York-Basel-Cambridge).

Monokotyle Pflanzen werden inzwischen routinemäßig mittels des biolistischen Ansatzes und mittels Agrobakterien transformiert (Komari et al., (1998); Advances in cereal gene transfer; Current Opinion in Plant Biotechnology 1, S. 161 ff.; Bilang et al. (1999), Transformation of Cereals, Genetic Engineering, 12, S. 113-148 Hrsg.: JK Setlow, Kluwer Academic / Plenum Publisher, New York). Weitere geeignete Methoden sind die elektrisch oder chemisch induzierte DNA-Aufnahme in Protoplasten, die Elektroporation von partiell permeabilisierten Zellen, die Makroinjektion von DNA in Blütenstände, die Mikroinjektion von DNA in Mikrosporen und Pro-Embryonen, die DNA-Aufnahme durch keimenden Pollen und die DNA-Aufnahme in Embryon durch Quellung (zur Übersicht: Potrykus, Physiol. Plant (1990), 269-273).

Darüber hinaus stellen die Protoplastentransformation, die Elektroporation von partiell permeabilisierten Zellen oder die Einbringung von DNA mittels Glasfasern alternative, dem Fachmann bekannte Verfahren dar.

Auch die erfolgreiche Transformation anderer Getreidearten wurde bereits beschreiben, z.B. für Gerste (Wan und Lemaux, s.o.; Ritala et al., s.o.; Krens et al., Nature 296 (1982), 72-74) und für Weizen (Becker et al., Plant J. (1994) 5 (2): 229-307; Nehra et al., Plant J. 5 (1994), 285-297).

Für Reis wurden unterschiedliche Transformationsmethoden beschrieben, wie z.B. die Agrobakterium-vermittelte Transformation (Hiei et al., Plant J. 6 (1994), 271-282; Hiei et al., Plant Mol. Biol. 35 (1997), 205-218; Park et al., J. Plant Biol. 38 (1995), 365-371), die Protoplasten-Transformation (Datta, In "Gene transfer to plants", Potrykus, Spangenberg (Eds.), Springer-Verlag, Berlin, Heidelberg, 1995, 66-75; Datta et al., Plant Mol. Biol. 20 (1992), 619-629; Sadasivam et al., Plant Cell Rep. 13 (1994), 394-396), der biolistische Ansatz zur Pflanzentransformation (Li et al., Plant Cell Rep. 12 (1993), 250-255; Cao et al., Plant Cell Rep. 11 (1992), 586-591; Christou, Plant Mol. Biol. (1997), 197-203) sowie die Elektroporation (Xu et al., In "Gene transfer to plants", Potrykus, Spangenberg (Eds.), Springer-Verlag, Berlin, Heidelberg, 1995; 201-208).

Ferner betrifft die vorliegende Erfindung auch das Vermehrungsmaterial und Erntegut der erfindungsgemäßen Pflanzen, das erfindungsgemäße Pflanzenzellen enthält. Der Begriff "Vermehrungsmaterial" umfaßt dabei jene Bestandteile der Pflanze, die geeignet sind zur Erzeugung von Nachkommen auf vegetativem oder generativem Weg. Für die vegetative Vermehrung eignen sich beispielsweise Stecklinge, Calluskulturen, Rhizome, Wurzelstöcke oder Knollen. Anderes Vermehrungsmaterial umfaßt beispielsweise Früchte, Samen, Sämlinge, Protoplasten, Zellkulturen etc. Vorzugsweise handelt es sich bei dem Vermehrungsmaterial um Knollen oder Samen.

Weiterhin betrifft die vorliegende Erfindung die Verwendung der erfindungsgemäßen Promotoren oder der mittels des erfindungsgemäßen Verfahrens identifizierten Promotoren zur karyopsenspezifischen Expression von Transgenen in Pflanzenzellen oder Pflanzen.

Außerdem betrifft die vorliegende Erfindung die Verwendung der erfindungsgemäßen Promotoren oder der mittels des erfindungsgemäßen Verfahrens identifizierten Promotoren zur karyopsenspezifischen Co-suppression von Genen oder Transgenen in Pflanzenzellen oder Pflanzen.

Der Begriff "Transgen" bedeutet dabei eine künstlich in eine Pflanze eingeführte DNA-Sequenz, die ein oder mehrere der erfindungsgemäßen Nukleinsäuremoleküle enthält.

Diese und andere Ausführungsformen sind dem Fachmann durch die Beschreibung und die Beispiele der vorliegenden Erfindung offenbart. Weiterführende Literatur zu den oben angeführten Methoden, Mitteln und Anwendungen, die im Sinne der vorliegenden Erfindung benötigt werden, ist dem Fachmann aus dem Stand der Technik bekannt. Zu diesem Zweck bieten sich unter anderem öffentliche Datenbanken an (z.B. "Medline"), die ggf. über Internet zur Verfügung stehen, z. B. unter der Adresse http://www.ncbi.nlm.nih.gov/PubMed/medline.html. Weitere Datenbanken und Adressen sind dem Fachmann geläufig und können aus dem Internet entnommen werden, z. B. unter der Adresse http://www.lycos.com. Eine Übersicht über Quellen und Informationen zu Patenten bzw. Patentanmeldungen in der Biotechnologie ist in Berks, TIBTECH 12 (1994), 352-364 gegeben.

Zur spezifischeren Beschreibung der Erfindung wird einer der erfindungsgemäßen Promotoren durch SEQ ID No.1 repräsentiert, bestehend aus 3.809 Basen der genomischen Sequenz des isolierten gbss1-Subklons p11/1 wie durch DSM 13398 hinterlegt. Darin enthalten sind 3.163 Basen des 5'-flankierenden Bereichs und 646 Basen des codierenden Bereichs der GBSS I. Vergleiche der in SEQ ID No.1 aufgeführten genomischen Sequenz mit dem isolierten cDNA-Klon der GBSS I (Block (1997) Dissertation, Universität Hamburg) zeigen im 5'untranslatierten Bereich an Position 2.333 bis 2436 eine Homologie von etwa 75 % und an Position 3.216 bis 3.262 eine Homologie von 100% mit dem cDNA-Klon. Der 5'untranslatierte Bereich des Gens wird durch ein ca. 670 Basen langes Leader-Intron (Position 2.436-3.101 in Seq ID No. 1) unterbrochen.

Der 5'flankierend vom Startcodon gelegene DNA-Bereich (Promotor und 5'untranslatierter Bereich mit Leader-Intron; SEQ ID. No. 1 Position 1-3.139) wurde hinsichtlich bekannter *cis*-regulatorischer DNA-Elemente von Pflanzen untersucht. Es wurden Endosperm- bzw. samenspezifische DNA-Elemente an folgenden Positionen im GBSS 1-Promotor (= SEQ ID No. 1) identifiziert:

| | |
|---|---|
| -300bp-Elemente (TGTAAAG) | Position 906 (-) TGHAAARK |
| | |
| RY repeat (CATGCATG) | Position 2138 (+) CATGCATG |
| | Position 929 (+) CATGCAT |
| | Position 989 (+) CATGCAT |
| | Position 270 (-) CATGCAT |
| | Position 2139 (-) CATGCAT |
| | |
| ACGT Motiv | Position 1346 (+) GTACGTG |
| | Position 1401 (+) GTACGTG |
| | Position 1836 (+) GTACGTG |
| | |
| Amylase-Box | Position 2488 (-) TATCCAT |
| | |
| E-Boxen (CANNTG) | Position 451 (+) CANNTG |
| | Position 942 (+) CANNTG |
| | Position 967 (+) CANNTG |
| | Position 987 (+) CANNTG |
| | Position 997 (+) CANNTG |
| | Position 1038 (+) CANNTG |
| | Position 1140 (+) CANNTG |
| | Position 1363 (+) CACGTG (G-Box) |
| | Position 1571 (+) CANNTG |
| | Position 1988 (+) CANNTG |
| | Position 2014 (+) CANNTG |
| | Position 2035 (+) CANNTG |
| | Position 2554 (+) CANNTG |
| | Position 3032 (+) CANNTG |
| | Position 1050 (-) CACGTG (G-Box) |
| | Position 1695 (-) CACGTG (G-Box) |
| | Position 2949 (-) CACGTG (G-Box) |
| | |
| Napin Motiv (TACACAT) | Position 308 (+) TACACAT |
| | Position 940 (+) TACACAT |
| | Position 264 (-) TACACAT |
| | |
| SEF4 Motiv | Position 330 (+) RTTTTTR |
| | Position 2868 (+) RTTTTTR |
| | Position 241 (+) RTTTTTR |
| | Position 639 (+) RTTTTTR |
| | Position 2878 (+) RTTTTTR |
| | Position 721 (-) RTTTTTR |
| | Position 2657 (-) RTTTTTR |
| | Position 3038 (-) RTTTTTR |

DNA-Elemente für eine pollenspezifische Genexpression wurden an folgenden Positionen gefunden:

| | |
|---|---|
| Pollen1 | Position 609 (+) AGAAA |
| (LAT52; L. esculentum) | Position 702 (+) AGAAA |
| | Position 1053 (+) AGAAA |
| | Position 1057 (+) AGAAA |
| | Position 1449 (+) AGAAA |
| | Position 3046 (+) AGAAA |
| | Position 27 (-) AGAAA |
| | Position 104 (-) AGAAA |
| | Position 141 (-) AGAAA |
| | Position 254 (-) AGAAA |
| | Position 409 (-) AGAAA |
| | Position 520 (-) AGAAA |
| | Position 559 (-) AGAAA |
| | Position 563 (-) AGAAA |
| | Position 656 (-) AGAAA |
| | Position 771 (-) AGAAA |
| | Position 822 (-) AGAAA |
| | Position 2707 (-) AGAAA |
| | Position 2812 (-) AGAAA |
| | Position 2819 (-) AGAAA |
| | Position 2923 (-) AGAAA |
| | |
| Q-Element (ZM 13) | Position 2842 (+) AGGTCA |
| | Position 2847 (+) AGGTCA |

DNA-Elemente, die an einer durch Zucker regulierten Genexpression beteiligt sind wurden an folgenden Positionen gefunden:

| | |
|---|---|
| TATCCAY-Motiv | Position 2488 (-) TATCCAY |
| | |
| CGACG-Element (AMY3, O. sativa) | Position 1761 (+) CGACG |
| | Position 1289 (-) CGACG |
| | Position 1488 (-) CGACG |
| | Position 1748 (-) CGACG |
| | Position 932 (-) CGACG |

Wurzelspezifische DNA-Elemente wurden an folgenden Positionen gefunden:

| | |
|---|---|
| Wurzel Motiv (Triticum aestivum POX1) | Position 63 (+) ATATT |
| | Position 278 (+) ATATT |
| | Position 501 (+) ATATT |
| | Position 753 (+) ATATT |
| | Position 890 (+) ATATT |
| | Position 277 (-) ATATT |
| | Position 304 (-) ATATT |
| | Position 870 (-) ATATT |

DNA-Elemente, die an einer hormonell regulierten Genexpression durch ABA beteiligt sind, wurden an folgenden Positionen gefunden:

| | |
|---|---|
| ABRE Motiv (Oryza sativa em) | Position 1347 (+) TACGTGTC |
| | Position 1067 (-) TACGTGTC |
| ABRE Motiv (Triticum aestivum L. Em) | Position 1930 (+) ACGTSSSC |
| DPBF Core (CDC3) | Position 941 (+) ACACNNG |
| | Position 951 (+) ACACNNG |
| | Position 966 (+) ACACNNG |
| | Position 996 (+) ACACNNG |
| | Position 1010 (+) ACACNNG |
| | Position 1025 (+) ACACNNG |
| | Position 1107 (+) ACACNNG |
| | Position 1570 (+) ACACNNG |
| | Position 1603 (+) ACACNNG |
| | Position 2077 (+) ACACNNG |
| | Position 296 (-) ACACNNG |

DNA-Elemente, die an einer hormonell regulierten Genexpression durch Auxin bzw. Ethylen beteiligt sind, wurden an folgenden Positionen gefunden:

| | |
|---|---|
| Auxin response factor (ARF A.thaliana) | Position 2984 (-) TGTCTC |
| | |
| NtBBF1 Motiv (roIB) | Position 614 (+) ACTTTA |
| | Position 793 (+) ACTTTA |
| | |
| Ethylen RE (L.esculentum4) | Position 3022 (+) AWTTCAAA |
| | Position 3028 (+) AWTTCAAA |

DNA-Elemente, die für eine Licht- oder Temperatur-regulierte Genexpression stehen, wurden an folgenden Positionen im GBSS I-Promotor gefunden:

| | |
|---|---|
| I-Box | Position 713 (-) GATAA |
| | Position 796 (-) GATAA |
| LowTemperature RE (H. vulgare) | Position 1019 (+) ACCGACA |
| LowTemperature RE (A.thaliana) | Position 1020 (+) CCGAC |
| | Position 1324 (+) CCGAC |
| | Position 1749 (-) CCGAC |
| | Position 2523 (-) CCGAC |

AT-reiche Regionen, wie sie aus verschiedenen anderen Promotoren als Enhancerelemente bekannt sind (J.E. Sandhu, 1998, Plant Mol. Biol. 37: 885-96) finden sich in dem durch SEQ ID No.1 repräsentierten Promotor an verschiedenen Stellen: Positionen 1-958, 1024-1213, 1912-1960 sowie 2527-3127. Ein basales DNA-Element, das für die Transkriptionsinitiantion wesentlich ist (TATA-Box), wurde an Position 2378 gefunden. 25 bp downstream der TATA-Box befindet sich nach Nikolov (D.B. Nikolov, 1997, PNAS 94: 15-22) der Transkriptionsinitiationspunkt. Neben anderen bekannten DNA-Motiven (CAAT-Box, GT1-Box, MART-Boxen, DOF-Boxen, Myb- und Myc-Boxen) enthält der unter SEQ ID No.1 aufgeführte Promotor weitere, bisher unbekannte Sequenzmotive. Ein DNA-Sequenzmotiv (CCACACACTACAA) an Position 2.283 zeigt Homologien zu DNA-Sequenzabschnitten des gbssl-Promotors aus Gerste und einem DNA-Bereich im Puroindolin-Promotor aus Weizen (Digeon et al. (1999) Plant Mol. Biol. 39: 1101-1112; Acc. No. AJ000548), der in Reis die Expression des GUS-Reportergens in Endosperm, Aleuronzellen und im Perikarp reguliert. Repeats der Sequenz (CA)ₙ befinden sich an den Positionen 948-956, 1.007-1.015 und 1.024-1.030. Ein sich wiederholendes Sequenzmotiv (CTCACC) befindet sich an den Positionen 1.259 und 1.267. Zwei direkte Sequenzwiederholungen (ACGTACGT) befinden sich an den Positionen 1.344 und 1.349. Weitere Sequenzwiederholungen (GAGAGC) befinden sich an Position 1.558, Position 1.614 (CGCGTG) und 1.644 (CCCACCGG). Ein Motiv der Sequenz (AAAC)₄. befindet sich an Position 1.887. Ein sich wiederholendes Motiv der Sequenz (GAA)ₙ befindet sich an den Positionen 2.321 und 2.379 bis 2.423. Sequenzbereiche, die Homologien zum GBSS I-Promotorbereich aus Gerste (GenBank Acc. No. X07931) aufweisen, befinden sich an den Positionen 1.383-1.406 (95% Sequenzidentität), 2.136-2.179 (93% Sequenzidentität) und 2.229-2.284 (90% Sequenzidentität).

### Hinterlegung von Mikroorganismen:

Das erfindungsgemäße Nukleinsäuremolekül gemäß SEQ ID No. 1 wurde bei der Deutschen Sammlung für Mikroorganismen und Zellkulturen (DSMZ) in Braunschweig, Deutschland gemäß Budapester Vertrag am 17. März 2000 (17.03.2000) durch Hinterlegung von Plasmid DNA offenbart:
Plasmid p11/1 enthaltend SEQ ID No. 1, Hinterlegungsnummer DSM 13398.

### Klonierungsverfahren

Zur Klonierung in *E.coli*-Bakterienstämme wurden die Vektoren pBluescript^{™} II SK(+/-) bzw. KS(+/-) Phagemid Vektoren (Stratagene GmbH, Heidelberg, Deutschland) und Lambda Fix^{®} II / Xhol Klonierungsvektor (Stratagene GmbH, Heidelberg, Deutschland)verwendet.

### Bakterienstämme

Für die Blueskript-Vektoren wurden die *E.coli*-Stämme DH5α (Life Technologies, Karlsruhe, Deutschland) und Epicurian Coli SURE^{®} (Stratagene GmbH, Heidelberg, Deutschland) verwendet. Für die Bakteriophagen-Vektoren wurde der Epicurian Coli Stamm XL1-Blue MRA (Stratagene) verwendet.

Für grundlegende molekularbiologische Arbeitstechniken wird auf Sambrook et al. 1989 verwiesen: Sambrook et al. (1989), Molecular Cloning; A Laboratory Manual, Second Edition; Cold Spring Harbour Laboratory Press).

### Ausführungsbeispiele

Die nachfolgenden Beispiele erläutern die Erfindung ohne sie in irgendeiner Hinsicht zu beschränken.

### 1. Herstellung der genomischen Weizenbank

Zur Herstellung der genomischen Weizenbanken wurde Gesamt-DNA aus etiolierten Keimlingen von Triticum aestivum L. cv. "Florida" isoliert. Zur Anzucht steriler etiolierter Keimlinge wurden reife Karyopsen für 20 min mit 1 % NaOCl, 0,1 % (v/v) Mucasol^{®} (Merz & Co., Frankfurt, Deutschland) inkubiert und anschließend 3x mit Aqua bidest gewaschen. Die Karyopsen wurden auf sterilem MS-Medium (Murashige & Skoog (1962), Physiol. Plant. 15: 473-479), dem 0,3% (w/v) GELRITE^{®} (Carl Roth GmbH & Co., Karlsruhe, Deutschland) zur Verfestigung zugesetzt wurde, ausgelegt. Das Wachstum erfolgte bei 26°C in Dunkelheit. Vierzehn Tage nach dem Plattieren wurden die Keimlinge abgeschnitten und in flüssigem Stickstoff eingefroren.

Der partielle Verdau der genomischen DNA erfolgte mit den Restriktionsenzymen BamH I bzw. Sau3A I (Life Technologies, Karlsruhe, Deutschland). Hierzu wurden 3 Aliquots die jeweils 100 µg genomische DNA enthielten mit 150 µl des entsprechenden Restriktionspuffers in einem Gesamtvolumen von 1,5 ml mit 12,5 Units, 6,25 Units bzw. 3,125 Units des Restriktionsenzyms BamH I bzw. 1,56 Units, 0,78 Units bzw. 0,39 Units Sau3A I für 1 h bei 37°C restringiert. Aliquots der partiell restringierten DNA wurden anschließend gelelektrophoretisch auf den Grad der Restriktion analysiert. Die Restriktionsenzyme wurden durch einmalige Phenol/Chloroform/ Isoamylalkohol (25:24:1, v/v) und Chloroform/Isoamylalkohol (24:1, v/v) Extraktion aus den Ansätzen entfernt. Abschließend wurde Saccharose bis zu einer Endkonzentration von 10% (w/v) zu jedem Ansatz zugegeben.

Die Größenfraktionierung der partiell restringierten DNA erfolgte in kontinuierlichen 10-40% Saccharose-Gradienten (w/v) (Sambrook *et al.* (1989)). Die einzelnen Aliquots der partiell restringierten DNAs wurde vor dem Beladen jeweils eines 15 ml Saccharosegradienten für 10 min auf 68°C erwärmt und dann auf 20°C abgekühlt. Die Zentrifugation der Gradienten erfolgte für 24 h, bei 20°C und 22000 rpm (Beckmann, Rotor SW 40). Nach der Zentrifugation wurden die einzelnen Zentrifugenröhrchen im Boden angestochen und jeweils 500 µl Aliquots gesammelt.

Von den einzelnen Fraktionen wurden 30 µl in einem 0,5%igen Agarosegel aufgetrennt und die Größenverteilung der DNA in den einzelnen Fraktionen bestimmt. Fraktionen, die genomische DNA von ca. 4,0 kb und größer enthielten, wurden vereinigt. Die Saccharose aus den Proben wurde durch Dialyse gegen Tris/EDTA-Puffer (10 mM/1mM) entfernt. Anschließend wurden die Proben mit 2-Butanol eingeengt und die DNA aus den Proben mit 2 Volumenteilen EtOH (99,8%)/ 2 M Ammoniumacetat (Endkonzentration) bei Raumtemperatur (RT) ausgefällt.

Zum Auffüllen der 3'Enden der partiell restringierten DNA wurden 20 µg der BamH I bzw. Sau3A I restringierten DNA mit 1 mM dATP, 1 mM dGTP (Roche, Mannheim), 6 µl 10x Pfu Reaktionspuffer und 10 Units native Pfu-DNA Polymerase (DNA-Polymerase mit "proof-reading" Aktivität; Stratagene GmbH, Heidelberg, Deutschland) in einem Endvolumen von 60 µl inkubiert. Die Reaktion wurde bei 72°C für 1 h 30 min durchgeführt. Anschließend erfolgte eine Phenol/Chloroform/Isoamylalkohol-, eine Chloroform/Isoamylalkohol-Extraktion der DNA und nachfolgend eine Präzipitation der DNA mit 1/10 Vol. 3M NaAc und 2,5 Vol. EtOH (absolut).

### 1.1. Ligation in Lambda Fix^{®} II/xho I Partial Fill-In Vektoren (Stratagene GmbH, Heidelberg, Deutschland)

Die BamH I bzw. Sau3A I restringierte genomische DNA wurde in den Lambda Fix^{®} II/Xho I Klonierungsvektor nach Angaben des Herstellers (Stratagene GmbH, Heidelberg, Deutschland) ligiert. Der Ligationsansatz enthielt: 1 µl des Lambda Fix^{®} II Vektors, 0,4 µg BamH I bzw. Sau3A I restringierte genomische DNA, 0,5 µl 10x Ligationspuffer, 2 Weiss Units T4 DNA-Ligase (MBI Fermentas GmbH, St. Leon-Rot, Deutschland); Weiss et al. (1968) J. Biol. Chem., 243: 4543-4555) in einem Endvolumen von 5 µl.

### 1.2. In vitro Verpackung der Ligationsprodukte

Zur Verpackung der Lambda Phagen wurde das *in vitro*-Verpackungskit "Gigapack^{®} II Gold" der Firma Stratagene (Stratagene GmbH, Heidelberg, Deutschland) verwendet und den Angaben des Herstellers gefolgt.
Von den Ligationsansätzen wurden jeweils 1 µl zu den Verpackungsansätzen dazugegeben und im Folgenden den Angaben des Herstellers gefolgt.

### 1.3. Anzucht der Bakterien zur Phagenvermehrung

Zur Phagenvermehrung wurde der *E.coli*-Bakterienstamm XL1-Blue MRA (P2) verwendet. Die Bakterien wurden in LB-Medium mit 10 mM MgSO₄, 0,2% (w/v) Maltose bis zu einer OD 600 = 0,5 bei 37°C, 180 rpm angezogen. Anschließend wurden die Bakterien bei 2000 rpm, 10 min, 4°C pellettiert und der Überstand verworfen. Das Bakterienpellet wurde in 10 mM MgSO₄ resuspendiert und die Bakteriendichte auf OD₆₀₀ = 0,5 eingestellt.

Zur Phagenvermehrung wurden aus den Verpackungsansätzen 1µl aus den Originalansätzen bzw. 1:10 Verdünnung der Originalansätze mit 200µl Bakteriensuspension (OD₆₀₀ = 0,5) gemischt und 15 min bei 37°C inkubiert. Anschließend wurden die einzelnen Ansätze mit 3 ml TOP-Agarose (48°C) gemischt und auf NZY-Festmedium nach Herstellerangaben (s.o. Lambda Fix^{®} II/xho I Partial Fill-In Vektoren, Stratagene) plattiert. Die Platten wurden für ca. 16 h bei 33°C inkubiert.

Die Phagentiter der Sau3A I bzw. der BamHI genomischen Banken wurden durch Auszählen der Phagenplaques bestimmt. Für die Sau3a I bzw. die BamHI Primärbanken wurden Phagentiter von 2,2 x 10⁷ pfu/ml bzw. 1,4 x 10⁷ pfu/ml ermittelt. Zur Bestimmung der durchschnittlichen Insertgrößen wurden von jeder Bank 10 einzelne Phagenklone amplifiziert, die Phagen-DNA isoliert (Sambrook *et al.* 1989) und die Insertgrößen nach Restriktionsverdau und gelelektrophoretischer Auftrennung ermittelt. Die durchschnittliche Insertgröße beträgt ca. 15,0 Kb für die BamH I bzw. 15,6 Kb für die Sau3A I Bank.

### 1.4. Amplifizierung der genomischen Banken

Zur Herstellung repräsentativer, amplifizierter genomischer Banken wurden von jeder Bank ca. 4,5 Millionen pfu plattiert. Die Ampflifizierung erfolgte nach den Angaben des Herstellers (Stratagene). Die Phagentiter der amplifizierten Banken betrug 6,3 x 10⁹ pfu/ml (BamHI-Bank) bzw. 2,0 x 10⁹ pfu/ml (Sau3A I-Bank).

### 2. Durchmustern der genomischen Banken

Die Identifizierung und Isolierung von Phagenklonen, deren genomische Inserts Sequenzen der gbssl-Gene tragen, erfolgte über Plaque-Hybridisierung. Zum Durchmustern der genomischen Banken wurden ca. 500.000 Phagen von jeder Bank ausplattiert. Das Ausplattieren der Phagen und Abziehen der Platten erfolgte nach Standardprotokollen (Sambrook *et al.,* 1989; Stratagene Lambda Fix^{®} II Manual). Als genspezifische Sonden wurden DNA-Fragmente von cDNA-Klonen der GBSS I (Block, M. (1997) "Isolierung, Charakterisierung und Expressionsanalysen von Stärkesynthase-Genen aus Weizen (Triticum aestivum L.)". Dissertation, Universität Hamburg) eingesetzt.

Die Markierung eines 283bp großen DNA-Fragments des gbssl cDNA-Klons erfolgte über eine spezifische PCR-Reaktion, unter Einbau von DIG-markierten dUTP's (Roche Diagnostics GmbH, Mannheim, Deutschland). Die PCR-Reaktion wurde mit Primern durchgeführt, die innerhalb des ersten Exons des gbssl cDNA-Klons (Position 146-429) liegen.
W1: 5'-ATGGCGGCTCTGGTCACGTC- 3' (SEQ ID No. 9)
W2: 5'-AGGCCGCCAGTCTTGCTCCA -3' (SEQ ID No. 10)

Der PCR-Reaktionsansatz setzte sich folgendermaßen zusammen:
10µl PCR-Puffer (1 0xconc.; Life Technologies)
3µl MgCl₂ (50mM; Life Technologies)
3µl DIG dUTPs (Roche Diagnostics GmbH, Mannheim)
3µl dNTP-Mix (je 5mM)
6µl Primer W1 (10pmol)
6µl Primer W2 (10pmol)
10ng Template (cDNA-Klon der gbssl)
1µl Taq-Polymerase (5U/µl; Life Technologies)
ad 100µl ddH₂O

Die Bedingungen für die PCR waren folgendermaßen:
I. 94°C, 5min
II. 94°C, 30sec
III. 62°C, 30sec
IV. 72°C, 60sec (IV.→ II. 29 Schleifen)
V. 72°C, 5min

Die Prähybridisierung der Filter erfolgte in 5x SSC, 3% Blockingreagenz (Boehringer Mannheim), 0,2% Na-dodecylsulfat (SDS), 0,1% N-Laurylsarcosin und 30µg/ml Heringssperma-DNA bei 65°C im Wasserbad. Die Hybridisierung mit den DIG-markierten DNA-Sonden (6ng/ml Hybridisierungslösung) erfolgte über Nacht bei 65°C im beschriebenen Standard-Hybridisierungspuffer. Alle weiteren Schritte der Chemilumineszenz-Reaktion mit CSPD^{®} erfolgten nach Angaben des Herstellers (Roche Diagnostics GmbH, Mannheim, Deutschland).

Positive Plaques wurden ausgestochen und über zwei weiteren Runden der Amplifikation und Plaque-Filterhybridisierung vereinzelt. Die DNA der isolierten positiven Phagen wurden gereinigt Qiagen^{®} Lambda Kit (Qiagen GmbH, Hilden, Deutschland), mit verschiedenen Restriktionsenzymen geschnitten und nach einer Agarose-Gelelektrophorese in Southern-Hybridisierungen mit den bereits beschriebenen Sonden analysiert.

3. Subklonierung der λ-Phagenklone in bakterielle Vektoren (pBlueskript^{™} II) Die genomischen Inserts der positiven Phagenklone wurden mit verschiedenen Restriktionsenzymen geschnitten. Die resultierenden Subfragmente wurden in bakterielle Vektoren (pBlueskript^{™} II SK(+/-) bzw. KS(+/-) Phagemid Vektoren; Stratagene GmbH, Heidelberg, Deutschland) kloniert.
Über Southern-Hybridisierungen erfolgte die Isolierung von gbssl-spezifischen Klonen mit 5'-stromaufwärts gelegenen regulativen Elementen.

### 4. Sequenzanalysen

Für die Sequenzierung der genomischen Klone der gbss I und ihrer 5'-stromaufwärts gelegenen regulativen Elemente wurde die Firma SeqLab GmbH (Göttingen) beauftragt.

### 5. Klonierungen von Promotor-Testvektoren

Die Funktionsfähigkeit der in SEQ ID No.1 aufgeführten 5'flankierenden DNA-Bereiche wurde in transienten und stabilen Expressionsanalysen überprüft. Als Reportergen wurde das Gen der β-Glucuronidase (GUS) verwendet (Jefferson (1987) Plant Molecular Biology Reporter Vol.5 (4): 387-405). Es wurden Promotortestvektoren kloniert, in denen die codierende Region des gus-Gens (uidA) unter der Kontrolle des in SEQ ID No.1 (Position 1-3.139) aufgeführten 5'flankierenden DNA-Bereichs steht. Die Klonierung erfolgte als transkriptionale Fusion. Zunächst wurde das uidA-Gen über einen partiellen Verdau zusammen mit dem nos-Terminator aus dem Vektor pCal-GUS (uidA-Gen unter Kontrolle des CaMV 35S-Promotors; Chris Warren, Stanford Universität, unveröffentlicht) herausgeschnitten und hinter die Multiple Cloning Site von pBluescript (Stratagene) kloniert. Der so erzeugte promotorlose Vektor (uidA-nos) wurde für die weiteren Klonierungen verwendet.

Auch die 5'-untranslatierte Leadersequenz einer mRNA kann einen Einfluß auf die gewebespezifische Expression eines Gens haben (Rouster et al.(1998) Plant J. 15 (3): 435-40). Die klonierten Promotortestvektoren beinhalten daher diesen Bereich des GBSS I-Gens. In der gewählten Klonierungsstrategie liegt das Start-Codon der β-Glucuronidase an der Position des Start-Codons der GBSS I.

### 5.1. Klonierung der gbss I-Promotortestvektoren

Das Ausganskonstrukt des gbss I-Promotortestvektors trägt etwa 7,5 kb des 5'flankierenden DNA-Bereiches der gbss I. Die Klonierung in den promotorlosen uidA-nos-Vektor erfolgte über Restriktionsverdau der Plasmide p11/1 (gbss1) und puidA-nos mit den Enzymkombinationen Nco I/ Xba I, Ncol/ Sac I und für einen partiellen Verdau mit Nco I / Sal I. Der 7,5 kb lange 5'flankierende Bereich wurde anschließend durch unterschiedliche Restriktionen verkürzt, was zur Entfernung von DNA-Bereichen führte, in denen einige der beschriebenen DNA-Elemente liegen. Der gbss I-Promotortestvektor wurde im 5'flankierenden Bereich durch Restriktionen mit den nachfolgend genannten Restriktionsenzymen deletiert. Es wurden auf diese Weise folgende Deletionskonstrukte des gbss 1-Promotors kloniert:
- 4,0 gbss I/gus (Sac I-Restriktion ca. 4 kb upstream des Startcodons von gbss1; enthält die Nukleotide 1-3139 von SEQ ID No. 1);
- 1,9 gbss l /gus (Xbal-Restriktion an Position 1240; enthaltend die Nukleotide 1241-3139 von SEQ ID No.1);
- 1,6 gbss I /gus (Smal-Restriktion an Position 1514; enthaltend die Nukleotide 1515-3139 von SEQ ID No:1);
- 1,3 gbss l /gus (Kpnl-Restriktion an Position 1826; enthaltend die Nukleotide 1827-3139 von SEQ ID No.1);
- 1,0 gbss l / gus (BamHI-Restriktion an Position 2176; enthaltend die Nukleotide 2177-3139 von SEQ ID No.1) und
- 0,4 gbss l / gus (Bgl II-Restriktion an Position 2727; enthaltend die Nukleotide 2692-3139 von SEQ ID No.1).

### 6. Transiente Expressionsanalysen der Promotor-Testvektoren

Die Funktionsfähigkeit der isolierten Promotorkonstrukte wurde in transienten Expressionsanalysen überprüft. Die Tests wurden mit den aus Beispiel 5 erhaltenen gbss 1-Promotortestvektoren und ihren Deletionskonstrukten durchgeführt.

Die transienten Expressionsanalysen erfolgten nach biolistischer Transformation von verschiedenen Geweben (Karyopsen, Embryonen, Blätter, Wurzeln) von Weizen. Die Transformation von Embryonen, Blätter und Wurzeln wurde nach Becker et al. (Plant J. (1994) 5 (2): 229-307) durchgeführt, während die biolistische Transformation des Endosperms von Karyopsen modifiziert nach Mena et al. (Plant J. (1998) 16(1), 53-62) erfolgte. Der Nachweis der Reportergenaktivität erfolgt durch histochemischen Nachweis der GUS-Aktivität (Jefferson (1987) Plant Molecular Biology Reporter Vol.5 (4): 387-405). Die Experimente an 10-30 Tage alten (dap), längs- und quergeschnittenen Weizenkaryopsen zeigten, daß der Promotor zur Expression des Reportergens im Endosperm führt. In den transienten Tests war die Aktivität des uidA-Reportergens unter Kontrolle des gbss1-Promotors relativ stark ausgeprägt.

6.1. Folgende Deletionskonstrukte des GBSS I-Promotors erwiesen sich in transienten Expressionsanalysen als funktionsfähig:
- 7,5 gbss I/ gus (enthält ca. 7,5 kb 'upstream' des Startcodons von gbss I; einschließlich der Nukleotide 1-3139 SEQ ID No.1)
- 4,0 gbss I / gus, enthaltend die Nukleotide 1-3139 von SEQ ID No.1)
- 1,9 gbss I / gus (Xbal-Restriktion an Position 1240)
- 1,6 gbss I / gus (Smal-Restriktion an Position 1514)
- 1,3 gbss I / gus (Kpnl-Restriktion an Position 1826)
- 1,0 gbss I / gus (BamHI-Restriktion an Position 2176)

Nach einer Deletion an Position 2.691 von SEQ ID No. 1 (-0,4 gbss I / gus) konnte keine GUS-Aktivität des Reportergens mehr festgestellt werden.

### 7. Stabile Transformation von Weizen mit den Promotor-Testvektoren

Die in Beispiel 5 beschriebenen Promotortestvektoren und Deletionskonstrukte wurden zur Erzeugung stabil transformierter Weizenpflanzen verwendet:
- 4,0 gbss I / gus (s.o.)
- 1,9 gbss I / gus (Xbal-Restriktion an Position 1240; SEQ ID No.1)
- 1,0 gbss I / gus (BamHI-Restriktion an Position 2176; SEQ ID No.1)

Die Herstellung der transgenen Pflanzen erfolgte nach der Methode von Becker et al. (Plant J. (1994) 5 (2): 229-307). Als Selektionsmarker wurden die glufosinate- bzw. Neomycin-Resistenz tragenden Plasmide p35S-PAT (Aventis CropScience GmbH, Frankfurt) bzw. pAct1 Dneo (Müller (1992) Dissertation, Universität Hamburg) eingesetzt.

### 8. Analyse der gus-Reportergenexpression in stabil transformierten Weizenpflanzen

Die funktionelle Analyse der gbss I- Promotoren erfolgte nach der Regeneration der transgenen Pflanzen und dem Nachweis einer stabilen und vollständigen Integration der Testkonstrukte in das Weizengenom über Southern-Analysen.

Die Reportergenaktivität in den regenerierten transgenen Pflanzen wurde über einen histochemischen GUS-Nachweis untersucht. Verschiedene Gewebe der Transgenen (Blätter, Wurzeln, Stengel, Endosperm, Embryo, Pollen) wurden analysiert. Die Karyopsen der mit den gbss I-Testvektoren stabil transformierten Pflanzen weisen eine starke GUS-Färbung im zentralen Stärkeendosperm auf. Die GUS-Aktivität konnte bereits in sehr jungen Karyopsen im sich entwickelnden Endosperm nachgewiesen werden. Sehr früh nach der Bestäubung ist außerdem eine Aktivität des gus-Reportegens im Perikarp nachweisbar, die in älteren Karyopsen nicht mehr auftritt. Keine GUS-Aktivität konnte dagegen im Embryo, dem Aleuron und der Embryo-umgebenden Region nachgewiesen werden. Auch im assimilierenden Gewebe der Blätter, sowie in den Stengeln und Wurzeln konnte keine Reportergen-Aktivität nachgewiesen werden. In transgenem Pollen wurde ebenfalls GUS-Aktivität detektiert. Quantitative Analysen der Expression des Reportergens erfolgten über fluorimetrische GUS-Nachweise sowie in Northern-Blot Analysen.

**Tabelle 1: Expressionsmuster der gbss 1-Promotorkonstrukte (vgl. Bsp. 5-7)**

| Gewebe | -4.0 GUS | -1.9 GUS | -1.0 GUS |
|---|---|---|---|
| Endosperm | | | |
| jung | ++ | ++ | + |
| alt | +++ | +++ | - |
| Perikarp | | | |
| jung | + | + | + |
| alt | - | - | - |
| Chlorophyllschicht | - | - | - |
| Embryo | - | - | - |
| Scutellum | - | + | + |
| Pollen | +++ | +++ | + |
| Blatt | - | - | - |

Hierbei zeigte sich, dass mit abnehmender Länge des in den Promotortestvektor integrierten Promotorfragments die Stärke der β-Glucuronidaseaktivität beziehungsweise der Reportergenexpression abnimmt. Eine Erklärung für diesen Effekt ist die Präsenz von AT-reichen Regionen, die bei der Verkürzung des Promotorbereiches schrittweise wegfallen. Diese Bereiche finden sich an den Positionen 1-958, 1024-1213 sowie 1912-1960 im Bereich des Konstrukts -4,0 gus, wobei die ersten zwei bei der Verkürzung zum Konstrukt -1,9 gus deletiert werden. Bei der weiteren Verkürzung zum Konstrukt -1,0 gus wird auch der AT-reiche Bereich 1912-1960 deletiert. Überraschend ist, dass auch das -1,0 gus-Konstrukt eine gewebespezifische Aktivität vermittelt, da in dieser Deletion von dem Bereich, der die TATA-Box 5'flankiert und in dem sich üblicherweise die cis-regulatorischen DNA-Elemente befinden, nur noch 38 bp vorhanden sind.

Northern Blot-Analysen zeigten die Expressionsmuster der unterschiedlichen Promotor-GUS-Konstrukte. Das uidA-Expressionsmuster im Verlauf der Karyopsenentwicklung der Pflanzen, die das Konstrukt -4,0 gus enthalten, entsprach sowohl im Endosperm als auch im Perikarp dem des gbss1-Gens. Auch das -1,9 GUS-Konstrukt und das -1,0 GUS-Konstrukt führten im Perikarp zu einem uidA-Expressionsmuster, das dem des gbss1-Gens entsprach. Für die Expression des uidA-Reportergens unter der Kontrolle dieser beiden Promotordeletionen im Endosperm war das Maximum der Aktivität weit nach hinten in der Karyopsenentwicklung verschoben, es lag etwa 25 Tage nach der Bestäubung oder später.

## Patentansprüche

1. Nukleinsäuremolekül mit der Funktion eines karyopsenspezifischen Promotors, das
a) die durch Seq ID No. 1 definierte oder die durch DSM 13398 (Plasmid p 11/1) hinterlegte Nucleinsäuresequenz umfaßt;
b) ein oder mehrere Sequenzelemente umfaßt, ausgewählt aus der Gruppe bestehend aus
i) cacgcaaagg cgcgtcggcc agccacgac (Seq ID No. 2);
ii) agaaacaaac aaacaaacaa aaaagt (Seq ID No. 3);
iii) cctttcagga cgatgcttcg gtgccttaag acacctacc tttgtgtcta tgacatgtga gcccaacag atggct (Seq ID No. 4);
iv) cccgtctagg cgttcggtgt ccggcc (Seq ID No. 5);
v) cagggagcct tcga (Seq ID No. 6);
vi) tcagccagtt ccaccccgtg cacg (Seq ID No. 7) und
vii) tactctggtc atgttaa (Seq ID No. 8);
c) einen funktionalen Teil der in a) genannten Nucleinsäuresequenz umfasst, wobei der funktionale Teil die Sequenzabschnitte 948-3139;1006-3139; 1240-3139; 1259-3139; 1382-3139; 1486-3139;1514-3139; 1655-3139; 1822-3139; 1887-3139; 2138-3139 oder 2176-3139 der Seq. ID No.1 umfasst; oder
d) eine Sequenz umfaßt, die mit einer der in a) genannten Nucleinsäuresequenzen zu etwa 90-99 % identisch ist.

2. Nukleinsäuremolekül nach Anspruch 1, der ein in Pflanzen aktiver Promotor ist.

3. Expressionskassette enthaltend ein Nukleinsäuremolekül nach einem oder mehreren der Ansprüche 1 - 2.

4. Vektor enthaltend ein Nukleinsäuremolekül nach einem oder mehreren der Ansprüche 1 - 2 oder eine Expressionskassette nach Anspruch 3.

5. Vektor nach Anspruch 4, der zur Transformation von pflanzlichen Zellen geeignet ist.

6. Wirtszelle, die genetisch modifiziert ist mit einer Expressionskassette nach Anspruch 3 oder einem Vektor nach einem oder mehreren der Ansprüche 4 - 5.

7. Wirtszelle nach Anspruch 6, die eine pro- oder eukaryontische Zelle ist.

8. Wirtszelle nach Anspruch 6, die eine Pflanzenzelle ist.

9. Pflanze enthaltend Pflanzenzellen nach Anspruch 8.

10. Vermehrungsmaterial oder Erntegut von Pflanzen nach Anspruch 9, enthaltend Pflanzenzellen nach Anspruch 8.

11. Verfahren zur Herstellung transgener Pflanzenzellen nach Anspruch 8, worin man Pflanzenzellen, -gewebe oder -teile oder Protoplasten mit einem Nukleinsäuremolekül nach einem oder mehreren der Ansprüche 1 - 2, einem Vektor nach einem oder mehreren der Ansprüche 4 - 5, mit einer Expressionskassette nach Anspruch 3 oder mit einer Wirtszelle nach Anspruch 6 transformiert, und die transformierten Pflanzenzellen, -gewebe, - teile oder Protoplasten in einem Wachstumsmedium kultiviert.

12. Verfahren zur Herstellung transgener Pflanzen nach Anspruch 9, worin man Pflanzenzellen, -gewebe oder -teile oder Protoplasten mit einem Nukleinsäuremolekül nach einem oder mehreren der Ansprüche 1-2, einem Vektor nach einem oder mehreren der Ansprüche 4-5, mit einer Expressionskassette nach Anspruch 3 oder mit einer Wirtszelle nach Anspruch 6 transformiert, die transformierten Pflanzenzellen, -gewebe, -teile oder Protoplasten in einem Wachstumsmedium kultiviert und daraus ganze Pflanzen regeneriert.

13. Verwendung eines Nukleinsäuremoleküls nach einem oder mehreren der Ansprüche 1-2 zur karyopsenspezifischen Expression von Genen in genetisch modifizierten Pflanzen.

14. Verwendung eines Nukleinsäuremoleküls nach einem oder mehreren der Ansprüche 1-2 zur karyopsenspezifischen Suppression von Genen in genetisch modifizierten Pflanzen.

15. Verfahren zur karyopsenspezifischen Genexpression in Pflanzen, worin ein Nukleinsäuremolekül gemäß einem oder mehreren der Ansprüche 1-2 stabil in das Genom einer Pflanzenzelle integriert wird und aus besagter Pflanzenzelle eine Pflanze regeneriert wird.

16. Verfahren zur karyopsenspezifischen Gensuppression in Pflanzen, worin ein Nukleinsäuremolekül gemäß einem oder mehreren der Ansprüche 1-2 stabil in das Genom einer Pflanzenzelle integriert wird und aus besagter Pflanzenzelle eine Pflanze regeneriert wird.

## Claims

1. A nucleic acid molecule with the function of a caryopsis-specific promoter, which nucleic acid molecule
a) comprises the nucleic acid sequence defined by Seq ID No. 1 or deposited by DSM 13398 (plasmid p 11/1);
b) comprises one or more sequence elements selected from the group consisting of
i) cacgcaaagg cgcgtcggcc agccacgac (Seq ID No.2);
ii) agaaacaaac aaacaaacaa aaaagt (Seq ID No. 3);
iii) cctttcagga cgatgcttcg gtgccttaag acacctacc tttgtgtcta tgacatgtga gcccaacag atggct (Seq ID No. 4);
iv) cccgtctagg cgttcggtgt ccggcc (Seq ID No. 5);
v) cagggagcct tcga (Seq ID No. 6);
vi) tcagccagtt ccaccccgtg cacg (Seq ID No. 7) and
vii) tactctggtc atgttaa (Seq ID No. 8);
c) comprises a functional portion of the nucleic acid sequence stated under a), the functional portion comprising the sequence segments 948 -3139; 1008-3139; 1240-3139; 1259-3139; 1382-3139; 1486-3139; 1514-3139; 1655-3139; 1822-3139; 1887-3139; 2138-3139 or 2176-3139 of Seq ID No. 1; or
d) comprises a sequence which has about 90-99% identity with one of the nucleic acid sequences stated under a).

2. A nucleic acid molecule as claimed in claim 1, which is a promoter active in plants.

3. An expression cassette comprising a nucleic acid molecule as claimed in one or more of claims 1-2.

4. A vector comprising a nucleic acid molecule as claimed in one or more of claims 1 - 2 or an expression cassette as claimed in claim 3.

5. A vector as claimed in claim 4 which is suitable for transforming plant cells.

6. A host cell which is genetically modified with an expression cassette as claimed in claim 3 or with a vector as claimed in one or more of claims 4 - 5.

7. A host cell as claimed in claim 6, which is a pro- or eukaryotic cell.

8. A host cell as claimed in claim 6, which is a plant cell.

9. A plant comprising plant cells as claimed in claim 8.

10. Propagation material or harvested material from plants as claimed in claim 9, comprising plant cells as claimed in claim 8.

11. A method of generating transgenic plant cells as claimed in claim 8, wherein plant cells, plant tissue, plant parts or protoplasts are transformed with a nucleic acid molecule as claimed in one or more of claims 1 - 2, a vector as claimed in one or more of claims 4 - 5, with an expression cassette as claimed in claim 3 or with a host cell as claimed in claim 6, and the transformed plant cells, plant tissues, plant parts or protoplasts are grown in a growth medium.

12. A method of generating transgenic plants as claimed in claim 9, wherein plant cells, plant tissue, plant parts or protoplasts are transformed with a nucleic acid molecule as claimed in one or more of claims 1 - 2, a vector as claimed in one or more of claims 4 - 5, with an expression cassette as claimed in claim 3 or with a host cell as claimed in claim 6, the transformed plant cells, plant tissues, plant parts or protoplasts are grown in a growth medium, and intact plants are regenerated from these.

13. The use of a nucleic acid molecule as claimed in one or more of claims 1-2 for the caryopsis-specific expression of genes in genetically modified plants.

14. The use of a nucleic acid molecule as claimed in one or more of claims 1-2 for the caryopsis-specific suppression of genes in genetically modified plants.

15. A method for the caryopsis-specific gene expression in plants, wherein a nucleic acid molecule as claimed in one or more of claims 1-2 is stably integrated into the genome of a plant cell, and the plant is regenerated from said plant cell.

16. A method for the caryopsis-specific gene suppression in plants, wherein a nucleic acid molecule as claimed in one or more of claims 1-2 is stably integrated into the genome of a plant cell, and a plant is regenerated from said plant cell.

## Revendications

1. Molécule d'acide nucléique qui assure la fonction d'un promoteur spécifique dans les caryopses,
a) qui comprend la séquence d'acide nucléique définie par la SEQ ID No:1 ou la séquence d'acide nucléique déposée par DSM 13398 (plasmide p11/1);
b) qui comprend un ou plusieurs éléments de séquence sélectionnés dans le groupe constitué de, la partie fonctionnelle comprenant les parties de séquence 948-3139; 1006-3139; 1240-3139; 1259-3139; 1382-3139; 1486-3139; 1514-3139; 1240-3139; 1259-3139; 7.382-3139; 1486-3139; 1514-3139; 1655-3139; 1822-3139; 1887-3139; 2138-3139 ou 2176-3139 de la SEQ ID No:1; ou
c) qui comprend une partie fonctionnelle de la séquence d'acide nucléique mentionnée en a)
d) qui comporte une séquence qui est identique à environ à l'une des séquences d'acide nucléique mentionnées en a).

2. Molécule d'acide nucléique selon la revendication 1, qui est un promoteur actif dans les plantes.

3. Cassette d'expression qui contient une molécule d'acide nucléique selon l'une ou plusieurs des revendications 1 et 2.

4. Vecteur qui contient une molécule d'acide nucléique selon l'une ou plusieurs des revendications 1 et 2 ou une cassette d'expression selon la revendication 3.

5. Vecteur selon la revendication 4 qui convient pour transformer des cellules végétales.

6. Cellule-hôte qui a été modifiée génétiquement par une cassette d'expression selon la revendication 3 ou un vecteur selon l'une ou plusieurs des revendications 4 et 5.

7. Cellule-hôte selon la revendication 6, qui est une cellule procaryote ou eucaryote.

8. Cellule-hôte selon la revendication 6, qui est une cellule végétale.

9. Plante qui contient des cellules végétales selon la revendication 8.

10. Matériau de multiplication ou produit de plantes selon la revendication 9, qui contient des cellules végétales selon la revendication 8.

11. Procédé de création de cellules transgéniques de plantes selon la revendication 8, dans lequel on transforme des cellules, des tissus ou des parties de plantes ou des protoplastes avec une molécule d'acide nucléique selon l'une ou plusieurs des revendications 1 et 2, un vecteur selon l'une ou plusieurs des revendications 4 et 5, une cassette d'expression selon la revendication 3 ou une cellule-hôte selon la revendication 6, et dans lequel on cultive dans un milieu de croissance les cellules, tissus, parties ou protoplastes de plantes.

12. Procédé de création de plantes transgéniques selon la revendication 9, dans lequel on transforme des cellules, des tissus ou des parties de plantes ou des protoplastes avec une molécule d'acide nucléique selon l'une ou plusieurs des revendications 1 et 2, un vecteur selon l'une ou plusieurs des revendications 4 et 5, une cassette d'expression selon la revendication 3 ou une cellule-hôte selon la revendication 6, et dans lequel on cultive dans un milieu de croissance les cellules, tissus, parties ou protoplastes de plantes transformées et dans lequel on régénère des plantes complètes à partir de ceux-ci.

13. Utilisation d'une molécule d'acide nucléique selon l'une ou plusieurs des revendications 1 à 2 pour l'expression, spécifique dans les caryopses, de gènes dans des plantes génétiquement modifiées.

14. Utilisation d'une molécule d'acide nucléique selon l'une ou plusieurs des revendications 1 et 2 pour l'inhibition, spécifique dans les caryopses, de gènes dans des plantes génétiquement modifiées.

15. Procédé d'expression, spécifique dans les caryopses, de gènes de plantes, dans lequel une molécule d'acide nucléique selon l'une ou plusieurs des revendications 1 et 2 est intégrée de manière stable dans le génome d'une cellule de plante et une plante est régénérée à partir de ladite cellule de plante.

16. Procédé pour l'inhibition, spécifique dans les caryopses, de gènes de plantes, dans lequel une molécule d'acide nucléique selon l'une ou plusieurs des revendications 1 et 2 est intégrée de manière stable dans le génome d'une cellule de plante et une plante est régénérée à partir de ladite cellule de plante.
